# EUROPEAN PATENT SPECIFICATION

(11) **EP 2 237 019 B1**
(45) Date of publication and mention of the grant of the patent: **27.04.2016**
(21) Application number: 10002880.2
(22) Date of filing: 18.03.2010
(51) Int. Cl.: G01N 21/51, G01N 15/14, G01N 21/53, G01N 21/64, G01N 33/487, G01N 33/49

(54) **Blood analyzer, method and computer program for determining existence and nonexistence of lymphoblasts in blood sample**
Blutanalysegerät, Verfahren und Computerprogramm zur Bestimmung des Vorliegens und Nichtvorliegens von Lymphoblasten in einer Blutprobe
Analyseur de sang, procédé et programme informatique pour déterminer l'existence et l'absence de lymphoblastes dans un échantillon de sang

(30) Priority: 31.03.2009 JP 2009087609
(43) Date of publication of application: 06.10.2010
(73) Proprietor: Sysmex Corporation, Kobe-shi, Hyogo 651-0073 (JP)
(72) Inventor: Kataoka, Yukiko, Kobe-shi, Hyogo 651-0073 (JP); Tsuji, Tomohiro, Kobe-shi, Hyogo 651-0073 (JP)
(74) Representative: Hoffmann Eitle

(56) References cited:
- EP-A1- 0 844 481
- EP-A1- 1 542 008
- EP-A1- 1 813 942
- EP-A2- 0 347 210
- EP-A2- 1 004 880
- EP-A2- 1 746 407
- US-A- 5 389 549
- US-A- 5 496 734
- US-A- 6 004 816

## Description

### FIELD OF THE INVENTION

The present invention relates to a blood analyzer for optically measuring a blood sample and classifying a cell group contained in the blood sample into plural populations. Further, the present invention relates to a method for determining the existence and nonexistence of lymphoblasts in a blood sample.

### BACKGROUND

In the blood plasma of peripheral blood, there are floating red blood cells, platelets, and white blood cells. Blood examination inspecting these cells can provide a variety of clinical information. For this reason, there have been examined a large number of samples. The blood examination is carried out using a hematocytometer. The hematocytometer provides automatic measurements of red blood cell counts, platelet counts, white blood cell counts, hemoglobin concentrations, and the like in the blood.

There are five types of white blood cells in normal peripheral blood, i.e., lymphocytes, monocytes, basophils, eosinophils, and neutrophils. Many hematocytometers have a function of classifying white blood cells in a blood sample into five types. Meanwhile, in diseases such as hematological malignancies, there is an appearance of cell types which are not present in the normal blood. For example, in acute lymphocytic leukemia (ALL), there is an appearance of a large number of lymphoblasts in the peripheral blood. Accordingly, the detection of lymphoblasts in the peripheral blood is very useful in the diagnosis of acute lymphocytic leukemia.

US 6004816 discloses a method for classification of white blood cells, including the steps of:
1) mixing a blood sample with a hemolytic agent which lyses red blood cells in the blood sample to such a degree as not to impede measurement, thereby bringing normal or abnormal blood cells to a state suitable for staining;
2) mixing the sample prepared in step 1) with a dye which is represented by a certain structural formula, and specifically binds to cellular RNA to increase in fluorescence intensity, thereby fluorescent-staining nucleated cells in the blood sample;
3) measuring an assay sample prepared in step 2) with a flow cytometer to measure scattered light and fluorescence; and
4) classifying normal white blood cells into at least 5 populations, and counting them, by the use of the intensities of the scattered light and the fluorescence measured in step 3).

This patent document 1 discloses definite separation, classification and counting of atypical lymphocytes from normal white blood cells.

JP-A-2007-263958 discloses a method for classification of blood cells, including classifying a differentiation and maturing stage of myelocytic cells and B lymphoid cells, using an antibody against a certain cellular marker. JP-A-2007-263958 discloses the classification of lymphoblasts, by the use of side-scattered light and a fluorescence-labeled CD45 antibody.

US 2005202400 discloses a method for classifying and counting white blood cells, which includes:
(1) a step of staining cells, with a dye which has specificity to cell nuclei, particularly DNA, or a dye which has specificity to RNA;
(2) a step of introducing the thus prepared sample into a flow cytometer;
(3) a step of measuring scattered light and fluorescence for the respective stained cells in the sample, and classifying white blood cells and coincidence cells/platelet clumps utilizing a difference in the intensity of a scattered light peak and a difference in the scattered light width; and
(4) a step of classifying and counting mature white blood cells, white blood cells with an abnormal DNA amount and immature white blood cells, utilizing a difference in the scattered light intensity and a difference in the fluorescence intensity of classified components.

The method for classifying white blood cells disclosed in US 6004816 can classify atypical lymphocytes from normal white blood cells and count them. However, the detection of lymphoblasts cannot be accomplished with this method.

The method for classification of blood cells disclosed in JP-A-2007-263958 requires the use of expensive fluorescence-labeled antibodies in measurements. As a consequence, there is a problem associated with increased measurement costs.

The method for classifying and counting white blood cells disclosed in US 2005202400 enables the classification and counting of white blood cells with an abnormal DNA amount, including lymphoblasts. However, cell types other than lymphoblasts are also included within the white blood cells with an abnormal DNA amount. Therefore, it is impossible to correctly detect whether or not lymphoblasts are present in the sample of interest.

EP 1 746 407 A2 discloses a blood analyzer comprising: a sample preparation section comprising a dispenser which provides a pre-determined amount of blood specimen, a reagent chamber containing a hemolytic agent, and mixing mechanism which mixes the hemolytic agent with the blood specimen to hemolyze red blood cells in the blood, whereby a measurement sample containing particles is prepared; a particle data acquirement section comprising a flow cell through which the measurement sample is to be flowed, a light source which irradiates a light to the measurement sample flowing through the flow cell, a first photo detector which detects a first optical information reflecting a particle character from the particles in the measurement sample to output a light intensity of the first optical information, and a second photo detector which detects a second optical information reflecting a particle character from the particles in the measurement sample to output a light intensity of the second optical information, wherein the particle data acquirement section acquires a particle data comprising data of the first and second light intensity associated with each particle; a data analysis section which counts white blood cells and determines whether malarial parasites are present or not based on the particle data acquired by the particle data acquirement section; and an output section which outputs the result of counting white blood cells and the determining result.

EP 0 844 481 A1 discloses a method for classifying and counting immature leukocytes comprising the steps of: (1) treating a hematological sample with a hemolytic agent which maintains immature leukocytes in a viable state and damages other leukocytes; (2) staining the damages leukocytes with a fluorochrome which can stain damaged cells; and (3) measuring at least one kind of fluorescence of the blood cells treated in the preceding step to classify and count leukocytes based on the intensities of the scattered light and the fluorescence.

### SUMMARY OF THE INVENTION

The scope of the present invention is defined solely by the appended claims, and is not affected to any degree by the statements within this summary.

The present invention has been made in view of the above-mentioned problems. That is, it is an object of the present invention to provide a blood analyzer as defined in appended claim 1, which enables the detection of lymphoblasts without the use of high-priced fluorescence-labeled antibodies. It is another object of the present invention to provide a method as defined in appended claim 5 for determining the existence and nonexistence of lymphoblasts in a blood sample, without the use of high-priced fluorescence-labeled antibodies. It is still another object of the present invention to provide a computer program as defined in appended claim 6 for determining the existence and nonexistence of lymphoblasts in a blood sample.

The blood analyzer of the present invention enables the detection of lymphoblasts without the use of fluorescence-labeled antibodies.

The determination method of the present invention enables the determination of the existence and nonexistence of lymphoblasts in a blood sample, without the use of fluorescence-labeled antibodies.

### BRIEF DESCRIPTION OF THE DRAWINGS

Fig. 1 is a perspective view illustrating an appearance of a blood analyzer according to an embodiment; Fig. 2 is a perspective view illustrating an appearance of a sample container; Fig. 3 is a perspective view illustrating an appearance of a sample rack;
Fig. 4 is a block diagram illustrating the configuration of a measurement unit according to an embodiment;
Fig. 5 is a schematic view illustrating an outline configuration of an optical detector;
Fig. 6 is a plan view illustrating the configuration of a sample transport unit according to the disclosure.
Fig. 7 is a front view illustrating the configuration of a first belt of a sample transport unit;
Fig. 8 is a front view illustrating the configuration of a second belt of a sample transport unit;
Fig. 9 is a block diagram illustrating the configuration of an information processing unit according to an embodiment;
Fig. 10 is a flowchart illustrating the operation procedure in a first measurement process of a blood analyzer according to an embodiment.
Fig. 11 is a flowchart illustrating the operation procedure in a second measurement process of a blood analyzer according to an embodiment.
Fig. 12 is a flowchart illustrating the processing procedure in a data processing process of a blood analyzer according to an embodiment.
Fig. 13A is a scattergram of side-scattered light intensity and side fluorescence intensity in the first measurement data.
Fig. 13B is a scattergram of forward-scattered light intensity and side fluorescence intensity in the first measurement data.
Fig. 14 is a scattergram of forward-scattered light intensity and side fluorescence intensity in the second measurement data.
Fig. 15A is a view illustrating an example of an analysis result screen of a blood analyzer according to an embodiment.
Fig. 15B is a view illustrating another example of an analysis result screen of a blood analyzer according to an embodiment.
Fig. 15C is a view illustrating a further example of an analysis result screen of a blood analyzer according to an embodiment.
Fig. 16A is a scattergram of forward-scattered light intensity and side fluorescence intensity in the first measurement data of a blood sample which contains lymphoblasts but does not contain nucleated red blood cells.
Fig. 16B is a scattergram of side-scattered light intensity and side fluorescence intensity in the first measurement data of a blood sample which contains lymphoblasts but does not contain nucleated red blood cells.
Fig. 17 is a scattergram of forward-scattered light intensity and side fluorescence intensity in the second measurement data of a blood sample which contains lymphoblasts but does not contain nucleated red blood cells.
Fig. 18A is a scattergram of forward-scattered light intensity and side fluorescence intensity in the first measurement data of a blood sample which contains nucleated red blood cells but does not contain lymphoblasts.
Fig. 18B is a scattergram of side-scattered light intensity and side fluorescence intensity in the first measurement data of a blood sample which contains nucleated red blood cells but does not contain lymphoblasts.
Fig. 19 is a scattergram of forward-scattered light intensity and side fluorescence intensity in the second measurement data of a blood sample which contains nucleated red blood cells but does not contain lymphoblasts.

### DETAILED DESCRIPTION OF THE PREFERRED EMBODIMENTS

The preferred embodiments of the present invention will be described hereinafter with reference to the drawings.

In this embodiment, there is provided a blood analyzer for detecting lymphoblasts in a blood sample, including mixing the blood sample with a nucleic acid-staining fluorescent dye to prepare an assay sample, and measuring the assay sample by an optical flow cytometer.

### [Configuration of blood analyzer]

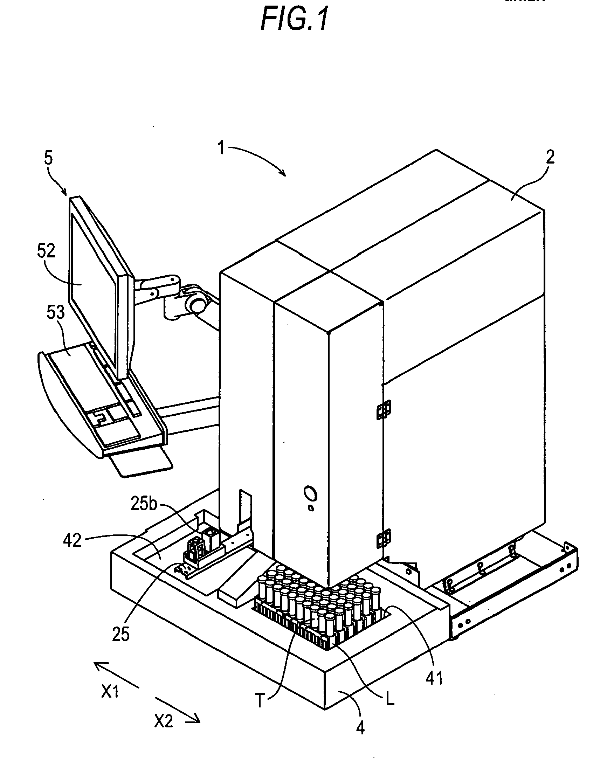

Fig. 1 is a perspective view illustrating an appearance of a blood analyzer according to this embodiment. The blood analyzer 1 according to this embodiment is a multi-item blood cell analyzing apparatus which detects blood cells, i.e., white blood cells, red blood cells, platelets and the like, which are contained in a blood sample, and counts each type of blood cells. As shown in Fig 1, the blood analyzer 1 is provided with a measurement unit 2, a sample transport unit 4 which is disposed on a front side surface of the measurement unit 2, and an information processing unit 5 which can control the measurement unit 2 and the sample transport unit 4.

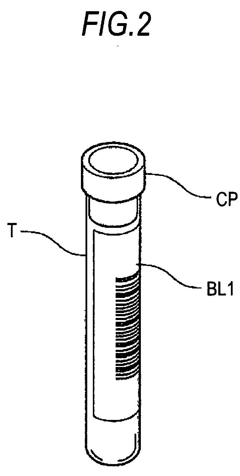

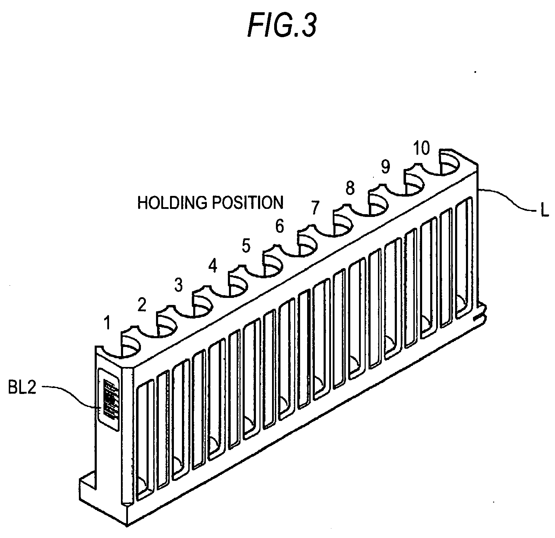

Fig. 2 is a perspective view illustrating an appearance of a sample container which contains a sample, and Fig. 3 is a perspective view illustrating an appearance of a sample rack which holds plural sample containers. As shown in Fig. 2, the sample container T is formed in a tubular shape, and an upper end thereof is opened. A blood sample gathered from a patient is contained in the sample container, and the opening on the upper end is sealed by a cap section CP. The sample container T is made of a transparent glass or synthetic resin, so that the blood sample therein is visible. In addition, the side surface of the sample container T is patched with a bar-code label BL1. A bar-code indicating the sample ID is printed on the bar-code label BL1. The sample rack L can arrange and hold 10 sample containers T. Each sample container T is held on in a vertical state (upright state) to the sample rack L. In addition, a bar-code label BL2 is patched on the side surface of the sample rack L. A bar-code indicating the rack ID is printed on the bar-code label BL2.

### <Configuration of Measurement Unit>

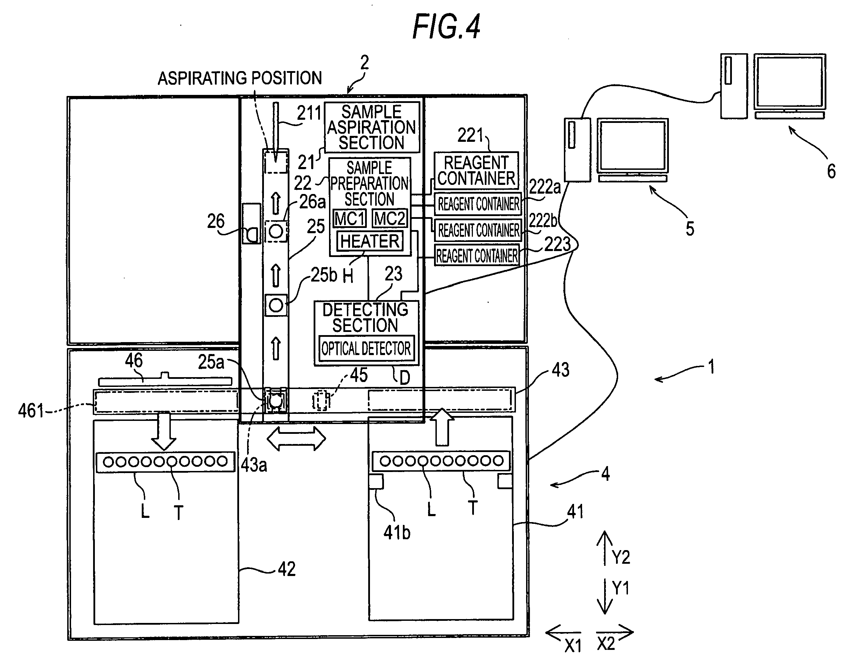

Next, the configuration of the measurement unit will be described. Fig. 4 is a block diagram illustrating the configuration of the measurement unit. As shown in Fig. 4, the measurement unit 2 includes a sample aspiration section 21 which aspirates blood as the sample from the sample container (blood collection tube) T, a sample preparation section 22 which prepares an assay sample to be used in measurements, from the blood aspirated by the sample aspiration section 21, and a detecting section 23 which detects blood cells from the assay sample prepared by the sample preparation section 22. In addition, the measurement unit 2 further includes a loading port (see Fig. 1) which is used to load the sample container T accommodated in the sample rack L, which is transported by a rack transport section 43 of the sample transport unit 4, into the measurement unit 2, and a sample container transport section 25 which loads the sample container T from the sample rack L into the measurement unit 2 and transports the sample container T up to an aspirating position by the sample aspiration section 21.

As shown in Fig. 4, the sample aspiration section 21 has an aspiration tube 211. In addition, the sample aspiration section 21 is provided with a syringe pump (not shown). In addition, the aspiration tube 211 can be vertically moved. The aspiration tube 211 is moved downward so that the aspiration tube 211 penetrates into the cap section CP of the sample container T transported to the aspirating position so as to aspirate the blood in the sample container.

The sample preparation section 22 is provided with a first mixing chamber MC1 and a second mixing chamber MC2. The aspiration tube 211 aspirates a given amount of a complete blood sample from the sample container T by a syringe pump (not shown). Then, the aspirated sample is transferred to positions of the first mixing chamber MC1 and the second mixing chamber MC2. Then, a given amount of the complete blood sample is dispensed and supplied to each of the chambers MC1 and MC2, by the syringe pump. In addition, the sample preparation section 22 is provided with a heater H for warming the first mixing chamber MC1 and the second mixing chamber MC2.

The sample preparation section 22 is connected via a tube to a reagent container 221 for accommodating a first reagent, a reagent container 222a for accommodating a second reagent, a reagent container 222b for accommodating a third reagent, and a reagent container 223 for accommodating a sheath fluid (diluent). Further, the sample preparation section 22 is connected to a compressor (not shown). The respective reagents can be aliquoted from the reagent containers 221, 222a, 222b, and 223, in response to a pressure generated by the compressor.

The first reagent is a reagent for detecting a blood cell group composed of lymphoblasts and nucleated red blood cells (hereinafter, referred to as "lymphoblast/nucleated red blood cell group"). The first reagent contains a hemolytic agent and a fluorescent dye. As the hemolytic agent contained in the first reagent, a known hemolytic agent may be employed which is used for measuring white blood cells. The use of the hemolytic agent results in damage to cell membranes of red blood cells and mature white blood cells, which contributes to shrinkage of the damaged blood cells. More specifically, the hemolytic agent contains a surfactant which damages cell membranes of red blood cells and mature white blood cells, and a solubilizing agent which reduces the size of damaged blood cells.

As the surfactant contained in the hemolytic agent, a nonionic surfactant is preferred. As the nonionic surfactant, a polyoxyethylene-based nonionic surfactant is preferred. As a specific polyoxyethylene-based nonionic surfactant, exemplified is a surfactant having the following structural formula (I):

R₁-R₂-(CH₂CH₂O)ₙ-H (I)

wherein
R₁ is a C₉-C₂₅ alkyl group, alkenyl group or alkynyl group,
R₂ is -O-, -COO- or 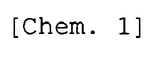 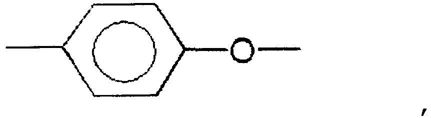 and
n is an integer of 10 to 40.

Specific examples of the surfactant represented by the structural formula (I) include polyoxyethylene(15) oleyl ether, polyoxyethylene(15) cetyl ether, polyoxyethylene(16) oleyl ether, polyoxyethylene(20) oleyl ether, polyoxyethylene(20) lauryl ether, polyoxyethylene(20) stearyl ether, polyoxyethylene(20) cetyl ether, and the like. In particular, polyoxyethylene(20) oleyl ether is preferred. Further, the hemolytic agent may contain one or more surfactants.

A concentration of the surfactant in the first reagent can be appropriately selected depending on the type of surfactants or the osmotic pressure of the hemolytic agent. For example, when the surfactant is polyoxyethylene oleyl ether, a concentration of the surfactant in the first reagent is in the range of 0.5 to 50.0 g/L, and preferably 1.0 to 20.0 g/L.

Examples of the solubilizing agent contained in the hemolytic agent include a sarcosine derivative, a cholic acid derivative, methylglucanamide, n-octyl β-glucoside, sucrose monocaprate, N-formylmethylleucylalanine and the like. Particularly preferred is the sarcosine derivative. In addition, the hemolytic agent may contain one or more solubilizing agents.

Examples of the sarcosine derivative may include a compound represented by the following structural formula (II) : 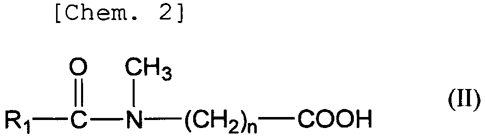 wherein R₁ is a C₁₀-C₂₂ alkyl group, and n is 1 to 5; and a salt thereof.

Specific examples of the sarcosine derivative may include sodium N-lauroylsarcosinate, sodium lauroyl methyl β-alanine, lauroylsarcosine, and the like. In particular, sodium N-lauroylsarcosinate is preferred.

Examples of the cholic acid derivative may include a compound represented by the following structural formula (III) : 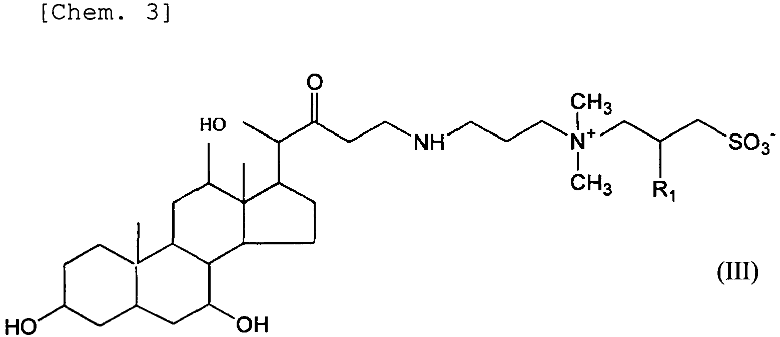 wherein R₁ is a hydrogen atom or a hydroxyl group; and a salt thereof.

Specific examples of the cholic acid derivative may include CHAPS (3-[(3-cholamidopropyl)dimethylammonio]-1-propanesulfonate ), CHAPSO (3-[(3-cholamidopropyl)dimethylammonio]-2-hydroxy-1-propan esulfonate), and the like.

Examples of the methylglucanamide may include a compound represented by the following structural formula (IV): 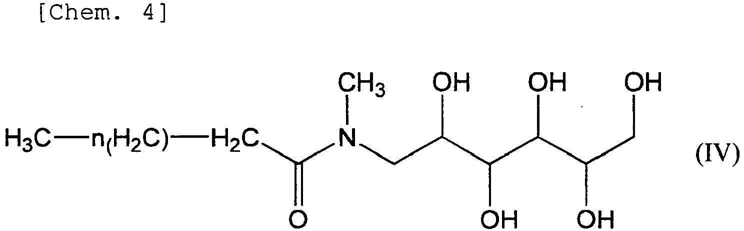 wherein n is 5 to 7.

Specific examples of the methylglucanamide may include MEGA8 (octanoyl-N-methylglucamide), MEGA9 (nonanoyl-N-methylglucamide), MEGA10 (decanoyl-N-methylglucamide), and the like.

A concentration of the solubilizing agent in the first reagent can be appropriately selected depending on the type of solubilizing agents to be used. For example, when a sarcosine derivative is used as the solubilizing agent, a concentration of the solubilizing agent in the first reagent is in the range of 0.05 to 3.0 g/L, and preferably 0.1 to 1.0 g/L. When a cholic acid derivative is used as the solubilizing agent, a concentration of the solubilizing agent in the first reagent is in the range of 0.1 to 10.0 g/L, and preferably 0.2 to 2.0 g/L. When a methylglucanamide is used as the solubilizing agent, a concentration of the solubilizing agent in the first reagent is in the range of 1.0 to 8.0 g/L, and preferably 2.0 to 6.0 g/L. When n-octyl β-glucoside, sucrose monocaprate, and N-formylmethylleucylalanine are used as solubilizing agents, a concentration of the solubilizing agent in the first reagent is in the range of 0.01 to 50.0 g/L, and preferably 0.05 to 30.0 g/L.

There is no particular limit to the fluorescent dye which is contained in the first reagent and is capable of staining a nucleic acid, as long as it is capable of fluorescent-staining the nucleic acid. The use of such a dye can stain nucleated blood cells, such as lymphoblasts having nucleic acid and nucleated red blood cells, while poorly staining red blood cells having no nucleic acid. Further, the nucleic acid-staining fluorescent dye can be appropriately selected by light irradiated from a light source. Specific examples of the nucleic acid-staining fluorescent dye may include propidium iodide, ethidium bromide, ethidium-acridine heterodimer, ethidium diazide, ethidium homodimer-1, ethidium homodimer-2, ethidium monoazide, trimethylene bis[[3-[[4-[[(3-methylbenzothiazol-3-ium)-2-yl]methylene]-1,4-dihydroquinolin]-1-yl]propyl]dimethylaminium]·tetraiod ide (TOTO-1), 4-[(3-methylbenzothiazol-2(3H)-ylidene)methyl]-1-[3-(trimethylam inio)propyllquinolinium-diiodide (TO-PRO-1), N,N,N',N'-tetramethyl-N,N'-bis[3-[4-[3-[(3-methylbenzothia zol-3-ium)-2-yl]-2-propenylidene]-1,4-dihydroquinolin-1-yl ]propyl]-1,3-propanediaminium-tetraiodide (TOTO-3), and 2-[3-[[1-[3-(trimethylaminio)propyl]-1,4-dihydroquinolin]-4-ylidene]-1-propenyl]-3-methylbenzothiazol-3-ium·diiodide (TO-PRO-3), and fluorescent dyes represented by the following structural formulae (V) to (XIII).

### <Structural formula (V)>

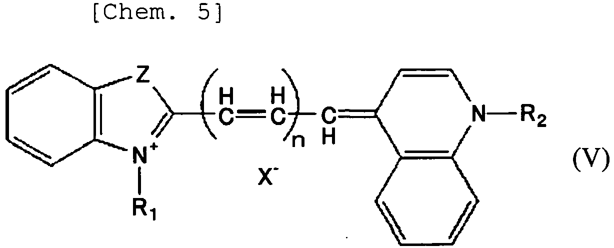

wherein R₁ and R₂ are each a lower alkyl group; n is 1 or 2; X⁻ is an anion; and Z is a sulfur atom, an oxygen atom, or a carbon atom substituted by a lower alkyl group.

The lower alkyl group of the structural formula (V) means a C₁-C₆ straight or branched alkyl group. Specific example of the lower alkyl group may include a methyl group, an ethyl group, a propyl group, a butyl group, an isobutyl group, a sec-butyl group, a tert-butyl group, a pentyl group, a hexyl group, and the like. In particular, methyl and ethyl groups are preferred. The Z is preferably a sulfur atom. The anion with regard to X⁻ includes halogen ions (fluorine, chlorine, bromine and iodine ions), boron halide ions (BF₄⁻, BCl₄⁻, BBr₄⁻, etc.), phosphorus compound ions, halooxy-acid ions, fluorosulfate ions, methyl sulfate ions, and tetraphenyl boron compound ions which have a halogen or halogeno-alkyl group as a substituent, in an aromatic ring. Particularly, iodine ions are preferred.

Among the compounds represented by the structural formula (V), a particularly preferable nucleic acid-staining fluorescent dye is NK-321 represented by the following structural formula.

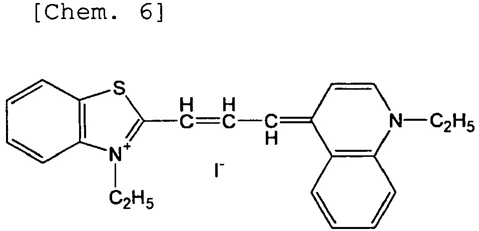

### <Structural formula (VI)>

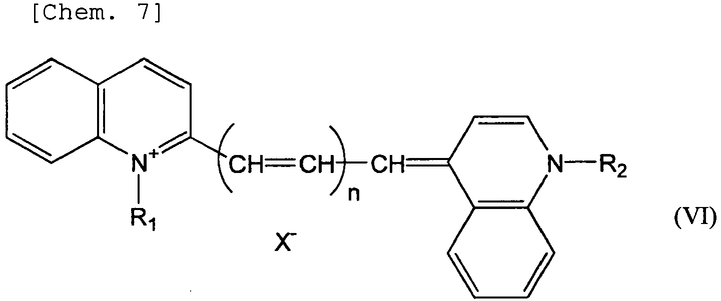

wherein R₁ and R₂ are each a lower alkyl group; n is 1 or 2; and X⁻ is an anion.

The lower alkyl group and the anion X⁻ in the structural formula (II) are as defined in the structural formula (I).

Among the compounds represented by the structural formula (VI), a particularly preferable nucleic acid-staining fluorescent dye is one represented by the following structural formula.

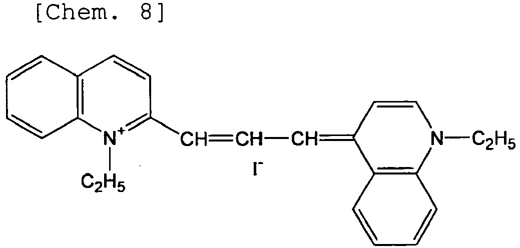

### <Structural formula (VII)>

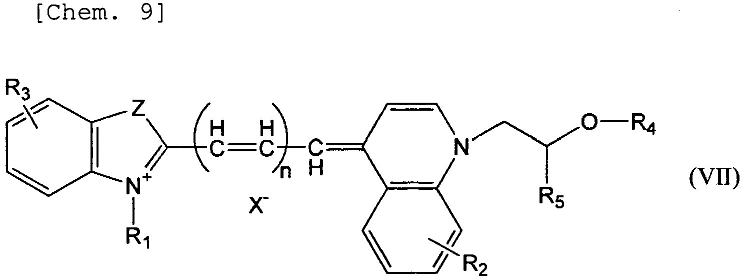

wherein R₁ is a hydrogen atom or a lower alkyl group; R₂ and R₃ are each a hydrogen atom, a lower alkyl group or a lower alkoxy group; R₄ is a hydrogen atom, an acyl group or a lower alkyl group; R₅ is a hydrogen atom or a lower alkyl group which may be substituted; Z is a sulfur atom, an oxygen atom, or a carbon atom which is substituted by a lower alkyl group; n is 1 or 2; and X⁻ is an anion.

The lower alkyl group and the anion X⁻ in the structural formula (VII) are as defined in the structural formula (V). The lower alkoxy group represents a C₁-C₆ alkoxy group. Specific examples of the lower alkoxy group may include methoxy, ethoxy, propoxy groups, and the like. In particular, methoxy and ethoxy groups are preferred. The acyl group is preferably an acyl group derived from an aliphatic carboxylic acid. Specific examples of the acyl group may include an acetyl group, a propionyl group, and the like. In particular, an acetyl group is preferred. Examples of the substituent of the lower alkyl group which may be substituted may include a hydroxyl group, and a halogen atom (fluorine, chlorine, bromine or iodine). The lower alkyl group which may be substituted may be substituted by 1 to 3 substituents. In particular, the lower alkyl group which may be substituted is preferably a lower alkyl group substituted by one hydroxyl group. Z is preferably a sulfur atom, and X⁻ is preferably a bromine ion or BF₄⁻.

Among the compounds represented by the structural formula (VII), particularly preferable nucleic acid-staining fluorescent dyes are represented by the following three structural formulae.

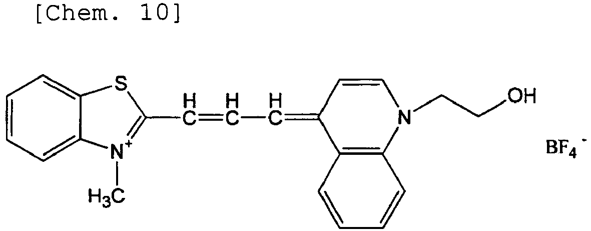

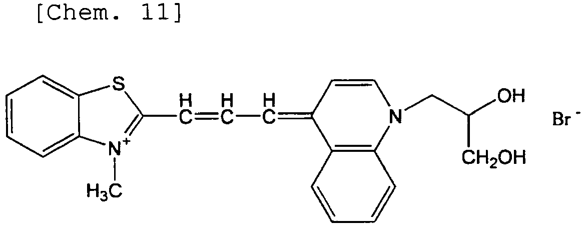

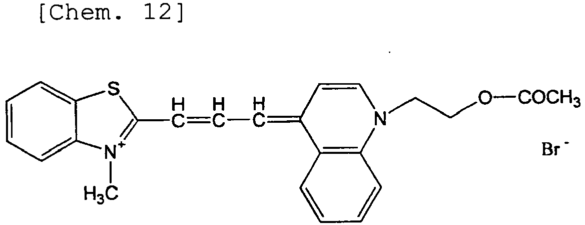

### <Structural formula (VIII)>

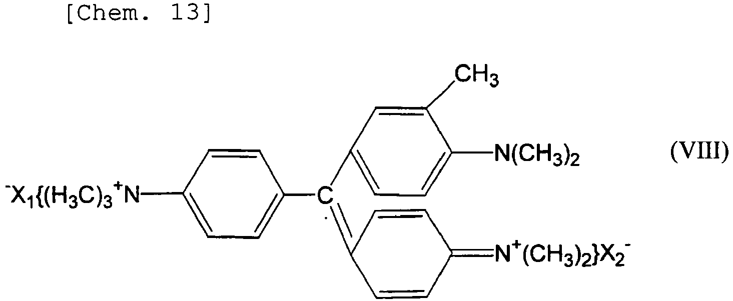

wherein X₁ and X₂ are independently Cl or I.

### <Structural formula (IX)>

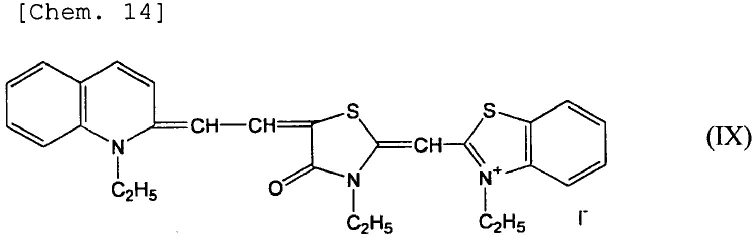

### <Structural formula (X)> (NK-1570)

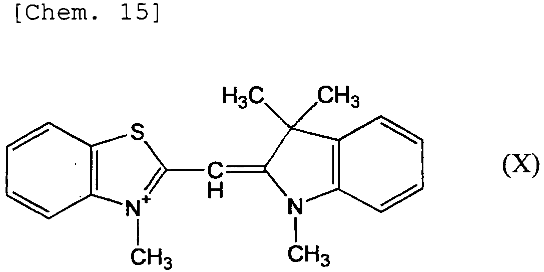

### <Structural formula (XI)> (NK-1049)

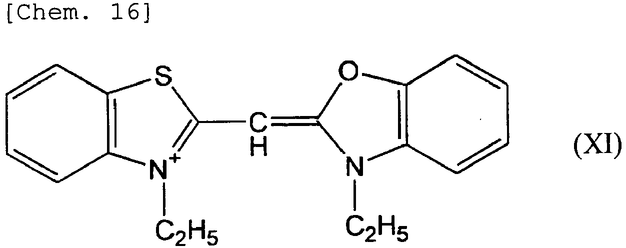

### <Structural formula (XII)> (NK-98)

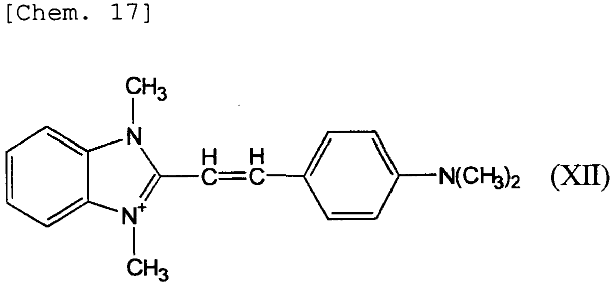

### <Structural formula (XIII)> (NK-141)

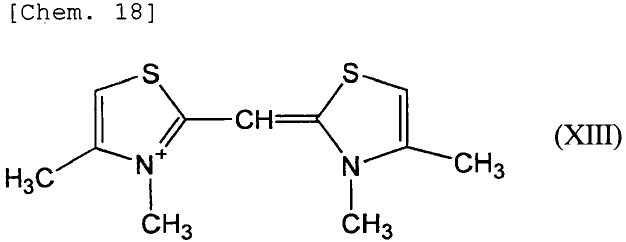

Among the above-exemplified nucleic acid-staining fluorescent dyes, a particularly preferable fluorescent dye contained in the first reagent is NK-321 represented by the following structural formula.

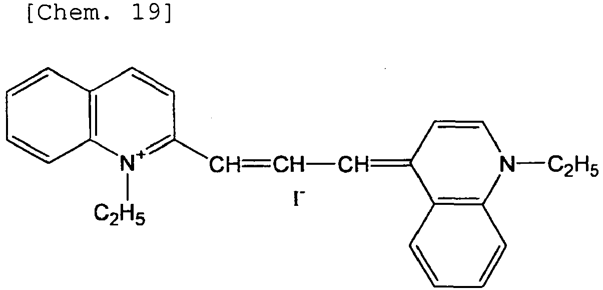

A concentration of the nucleic acid-staining fluorescent dye in the first reagent may be in the range of 10 to 500 mg/L. Particularly preferred is in the range of 30 to 100 mg/L. Further, the first reagent may contain one or more nucleic acid-staining fluorescent dyes.

A pH of the first reagent may be in the range of 5.0 to 9.0. Preferred is a pH of 6.5 to 7.5. Particularly preferred is a pH of 6.8 to 7.3. The pH of the first reagent may be adjusted by a buffer or a pH-adjusting agent. Examples of the buffer may include a Good buffer such as HEPES, 3-morpholinopropanesulfonic acid (MOPS) or 2-hydroxy-3-morpholinopropanesulfonic acid (MOPSO), a phosphate buffer, and the like. Examples of the pH-adjusting agent may include sodium hydroxide, hydrochloric acid, and the like.

An osmotic pressure of the first reagent can be appropriately set depending on the type of the above-mentioned surfactants or the concentration thereof in the first reagent. A specific osmotic pressure of the first reagent may be in the range of 10 to 600 mOsm/kg. Further, an osmotic pressure of the first reagent may be adjusted by adding sugars, amino acids, sodium chloride or the like to the first reagent. Specific examples of the sugars may include monosaccharide, polysaccharide, sugar alcohol and the like. For the monosaccharide, glucose or fructose is preferred. For the polysaccharide, arabinose is preferred. For the sugar alcohol, xylitol, sorbitol, mannitol, or ribitol is preferred. As the sugar which is added to the first reagent, preferred is a sugar alcohol, particularly xylitol. When xylitol is added to the first reagent, a concentration of xylitol in the first reagent is preferably in the range of 1.0 to 75.0 g/L, and particularly preferably 20.0 to 50.0 g/L. Specific examples of the amino acid may include valine, proline, glycine, alanine, and the like. In particular, preferred is glycine or alanine. When glycine is added to the first reagent, a concentration of glycine in the first reagent is preferably in the range of 1.0 to 50.0 g/L, and particularly preferably 10.0 to 30.0 g/L.

An electric conductivity of the first reagent is preferably in the range of 0.01 to 3 mS/cm. Particularly preferred is in the range of 0.1 to 2 mS/cm. Further, a chelating agent, a preservative or the like may be added to the first reagent. As the chelating agent, exemplified is EDTA-2K, EDTA-3Na, or the like. As the preservative, exemplified is Proxel GXL (manufactured by Avecia), material TKM-A (manufactured by API Corporation), or the like.

The second reagent is a hemolytic agent for the measurement of nucleated red blood cells (NRBC). Examples of the hemolytic agent for the measurement of NRBC may include Stromatolyser NR hemolytic reagent (manufactured by Sysmex Corporation). The third reagent is a staining solution for the measurement of NRBC. Examples of the staining solution for the measurement of NRBC may include Stromatolyser NR dye solution (manufactured by Sysmex Corporation). The fourth reagent is a sheath fluid which is supplied to a sheath flow cell which will be illustrated hereinafter. The sheath fluid is also used as a diluent. For example, the sheath fluid may be Cellpack (II) (manufactured by Sysmex Corporation).

The detecting section 23 includes an optical detector D which is capable of performing WBC measurement (white blood cell counting) and DIFF measurement (white blood cell classification). The optical detector D is configured such that the detection of WBC (mature white blood cells), NRBC (nucleated red blood cells), and lymphoblasts (L-Blast) can be performed by a flow cytometry method using semiconductor lasers. By using the detecting section 23, it is possible to achieve five classifications of white blood cells (WBC) into neutrophils (NEUT), lymphocytes (LYMPH), eosinophils (EO), basophils (BASO) and monocytes (MONO). When it is desired to measure a lymphoblast/nucleated red blood cell group, an assay sample (L-Blast assay sample), which is a mixture of a blood sample and a first reagent, is supplied to the optical detector D. When it is desired to measure NRBC, an assay sample (NRBC assay sample), which is a mixture of a blood sample, a second reagent and a third reagent, is supplied to the optical detector D.

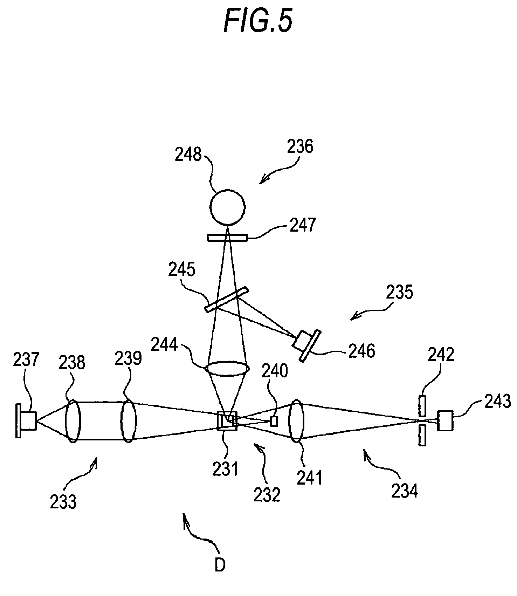

Fig. 5 shows the outline configuration of the optical detector D. The optical detector D sends an assay sample and a sheath fluid into a flow cell 231, and generates a liquid current in the flow cell 231. In addition, the optical detector D measures optical information by irradiating the blood cells included in the liquid current passing through the flow cell 231 with a semiconductor laser light. The optical detector D includes a sheath flow system 232, a beam spot-forming system 233, a forward-scattered light receiving system 234, a side-scattered light receiving system 235, and a side-fluorescent light receiving system 236.

The sheath flow system 232 is configured such that the assay sample flows in the flow cell 231 in a state of being surrounded by a sheath fluid. The beam spot-forming system 233 is configured such that the light irradiated from a semiconductor laser 237 passes through a collimator lens 238 and a condenser lens 239 so as to irradiate the flow cell 231. In addition, the beam spot-forming system 233 is provided with a beam stopper 240.

The forward-scattered light receiving system 234 is configured such that the forward-scattered light is condensed by a forward-condensing lens 241, and the light passing through a pin hole 242 is received by a photodiode (forward-scattered light receiving section) 243.

The side-scattered light receiving system 235 is configured such that the side-scattered light is condensed by a side-condensing lens 244, and a part of the light is reflected on a dichroic mirror 245 so as to be received by a photodiode (side-scattered light receiving section) 246.

Light scattering is a phenomenon occurring such that particles such as blood cells act as an obstacle to light in the advancing direction thereof, and the light is changed in the advancing direction by the particles. By detecting the scattered light, information on the size or material of the particle can be obtained. In particular, the information on the size of the particle (blood cell) can be obtained from the forward-scattered light. In addition, the information on the content of the particle can be obtained from the side-scattered light. When a laser light is irradiated to the blood cell particle, the side-scattered light intensity depends on the complexity of the inside of the cell (shape, size, density, or granulated amount of nucleus). Therefore, these scattered light intensities can be used in the measurement of a lymphoblast/nucleated red blood cell group, the measurement of nucleated red blood cells, the classification of white blood cells, and the like.

The side-fluorescent light receiving system 236 is configured such that the light that passed through the dichroic mirror 245 further passes through a spectral filter 247 and is received by an avalanche photodiode (fluorescence receiving section) 248.

When light is irradiated to blood cells stained with a fluorescent material, light is generated of which the wavelength is longer than that of the irradiated light. If staining is sufficiently performed, the fluorescence intensity becomes stronger. By measuring the fluorescence intensity, the information on the staining degree of the blood cell can be obtained. Therefore, differences in the (side) fluorescence intensity can be used in the measurement of a lymphoblast/nucleated red blood cell group, the measurement of nucleated red blood cells, the classification of white blood cells, and the like.

Returning to Fig. 4, the configuration of the sample container transport section 25 will be described. The sample container transport section 25 is provided with a hand section 25a which can grasp the sample container T. The hand section 25a is provided with a pair of grasping members which are disposed so as to face each other. The grasping members can be close to or away from each other by the action of the hand section 25a. The grasping members can grasp the sample container T by being close to each other in a state where the sample container T is interposed therebetween. In addition, the sample container transport section 25 can move the hand section 25a in a vertical direction and in a backward or forward direction (Y direction), and can also oscillate the hand section 25a. Thereafter, the sample container T which is accommodated in the sample rack L and positioned at the sample supply position 43a is grasped by the hand section 25a. In this state, the hand section 25a moves upward, so that the sample container T is pulled out of the sample rack L. Then, the hand section 25a is oscillated, so that the sample in the sample container T is stirred.

In addition, the sample container transport section 25 is provided with a sample container setting section 25b which includes a hole section through which the sample container T can be inserted. The sample container T grasped by the above-mentioned hand section 25a moves after the stirring is completed. Then, the grasped sample container T is inserted into the hole section of the sample container setting section 25b. Thereafter, the grasping members are away from each other, so that the sample container T is released from the hand section 25a, whereby the sample container T is set in the sample container setting section 25b. The sample container setting section 25b can horizontally move in the Y1 and Y2 directions in the drawing by a driving force of a stepping motor (not shown).

In the measurement unit 2, a bar-code reading section 26 is provided. The sample container setting section 25b can move to a bar-code reading position 26a near the bar-code reading section 26 and an aspirating position 21a carried out by the sample aspiration section 21. When the sample container setting section 25b moves to the bar-code reading position 26a, the set sample container T is horizontally rotated by a rotation mechanism (not shown) and the sample bar-code is read by the bar-code reading section 26. Accordingly, even when the bar-code label BL1 of the sample container T is positioned on the opposite side with respect to the bar-code reading section 26, the bar-code label BL1 can face the bar-code reading section 26 by rotating the sample container T, whereby the bar-code reading section 26 can definitely read the sample bar-code. In addition, when the sample container setting section 25b is moved to the aspirating position, the sample is aspirated from the set sample container T by the sample aspiration section 21.

### <Configuration of Sample Transport Unit>

Next, the configuration of the sample transport unit 4 will be described. As shown in Fig. 1, the sample transport unit 4 is disposed in front of the measurement unit 2 of the blood analyzer 1. The sample transport unit 4 can transport the sample rack L in order to supply the sample to the measurement unit 2.

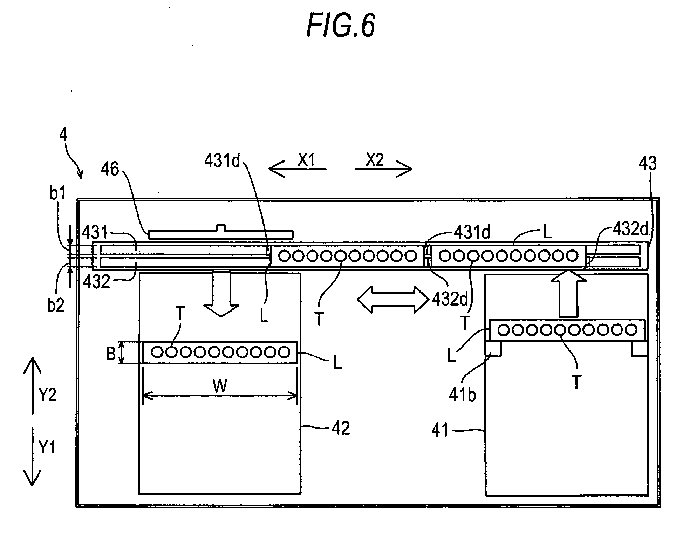

Fig. 6 is a plan view illustrating the configuration of the sample transport unit 4. As shown in Fig. 6, the sample transport unit 4 is provided with: a before-analysis rack holding section 41 which can hold the plural sample racks L each holding a sample container T which accommodates samples before analysis is carried out thereon; an after-analysis rack holding section 42 which can hold the plural sample racks L each holding a sample container T in which the sample is aspirated by the measurement unit 2; and a rack transport section 43 which horizontally moves the sample rack L in a straight line in the direction of arrow X1 or X2 in the drawing in order to supply the sample to the measurement unit 2 and transports the sample rack L received from the before-analysis rack holding section 41 to the after-analysis rack holding section 42.

The before-analysis rack holding section 41 has a quadrangular shape in plane view, and the width thereof is slightly larger than the width of the sample rack L. The before-analysis rack holding section 41 is formed to be lower by one stage than the surrounding surface. On an upper face of the before-analysis rack holding section 41, the before-analysis sample racks L are disposed. In addition, the rack sending sections 41b are provided in both faces of the before-analysis rack holding section 41 so as to be protruded inward. The rack sending sections 41b protrude so as to contact the sample rack L. In this state, the rack sending sections are moved backward (a direction so as to be closer to the rack transport section 43) and thus the sample rack L is moved backward. The rack sending sections 41b are configured to be driven by a stepping motor (not shown) which is provided below the before-analysis rack holding section 41.

As shown in Fig. 6, the rack transport section 43 can move the sample rack L sent by the before-analysis rack holding section 41 in the X direction as described above. On the transport path of the sample rack L by the rack transport section 43, there is a sample supply position 43a for supplying a sample to the measurement unit 2, as shown in Fig. 4. The sample transport unit 4 is controlled by the information processing unit 5 and transports the sample to the sample supply position 43a. Then, the hand section 25a of the measurement unit 2 grasps the transported sample container T and takes out the sample container T from the sample rack L, thereby completing a supply of the sample. When the sample container T is returned to the sample rack L from the measurement unit 2, the rack transport section 43 is waiting for transportation from when the sample container T was received. Or, the sample rack L is transported to another position, and then the sample rack L is transported such that a holding position, which was empty by the sample container T being received in the measurement unit 2, is positioned at the sample supply position 43a. Accordingly, in a state where the holding position into which the sample container T was not inserted is positioned in the sample supply position 43a, the hand section 25a can definitely return the sample container T to the sample rack L.

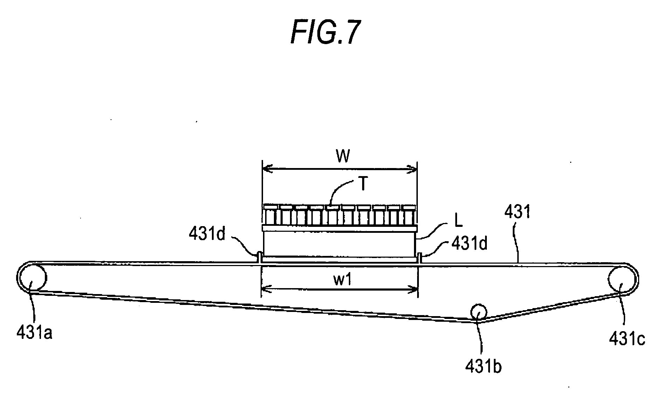

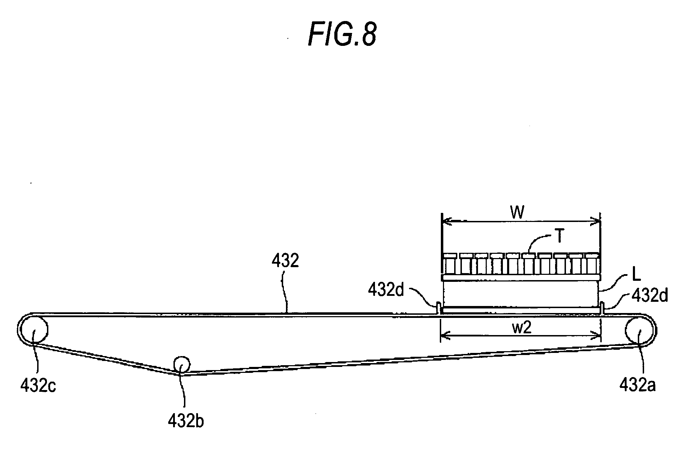

In addition, as shown in Fig. 6, the rack transport section 43 has two independently operable belts, that is, a first belt 431 and a second belt 432. Widths b1 and b2 in the direction of arrow Y of the first belt 431 and the second belt 432 are respectively equal to or less than half of a width B in the direction of arrow Y of the sample rack L. The first belt 431 and the second belt 432 are disposed in parallel so as not to protrude from the width B of the sample rack L when the rack transport section 43 transports the sample rack L. Fig. 7 is a front view illustrating the configuration of the first belt 431, and Fig. 8 is a front view illustrating the configuration of the second belt 432. As shown in Figs. 7 and 8, the first belt 431 and the second belt 432 are annularly formed. The first belt 431 is disposed so as to surround rollers 431a to 431c and the second belt 432 is disposed so as to surround rollers 432a to 432c. In the outer peripheral section of the first belt 431, two protrusions 431d are provided so as to have an inner width w1 slightly larger (for example, 1 mm) than a width W in the X direction of the sample rack L. Similarly, in the outer peripheral section of the second belt 432, two protrusions 432d are provided so as to have the same inner width w2 as the inner width w1. The first belt 431 is configured such that the sample rack L held inside of the two protrusions 431d is moved in the direction of arrow X by being moved along the outer peripheries of the rollers 431a to 431c by a stepping motor (not shown). The second belt 432 is configured such that the sample rack L held inside of the two protrusions 432d is moved in the direction of arrow X by being moved along the outer peripheries of the rollers 432a to 432c by a stepping motor (not shown). In addition, the first belt 431 and the second belt 432 are configured so as to move the sample rack L independently of each other.

As shown in Fig. 4, a sample container sensor 45 is provided on the transport path of the rack transport section 43. The sample container sensor 45 is a contact sensor. The sample container sensor 45 includes a curtain-like contact piece, a light-emitting element for emitting light, and a light-receiving element (not shown). The sample container sensor 45 is configured such that the contact piece is bent when brought into contact with a substance to be detected which is a detection object and the light emitted from the light-emitting element is thus reflected by the contact piece and enters the light-receiving element. Accordingly, while the sample container T which is a detection object accommodated in the sample rack L passes under the sample container sensor 45, the contact piece is bent by the sample container T and the sample container T can be detected.

As shown in Fig. 4, a rack delivery section 46 is disposed so as to face the after-analysis rack holding section 42 which will be illustrated hereinafter, with the rack transport section 43 therebetween. The rack delivery section 46 is configured to horizontally move in a straight line in the direction of arrow Y by a driving force of a stepping motor (not shown). Therefore, when the sample rack L is transported to a position 461 (hereinafter, referred to as "after-analysis rack delivery position") between the after-analysis rack holding section 42 and the rack delivery section 46, the sample rack L can be pushed and moved into the after-analysis rack holding section 42 by moving the rack delivery section 46 toward the after-analysis rack holding section 42.

The after-analysis rack holding section 42 has a quadrangular shape in plane view, and the width thereof is slightly larger than the width of the sample rack L. The after-analysis rack holding section 42 is formed to be lower by one stage than the surrounding surface. On an upper face of the after-analysis rack holding section 42, the analyzed sample racks L are held. The after-analysis rack holding section 42 is connected to the above-mentioned rack transport section 43. And, as described above, the sample rack L is transported from the rack transport section 43 by the rack delivery section 46.

According to the configuration as described above, the sample transport unit 4 moves the sample rack L disposed on the before-analysis rack holding section 41 to the rack transport section 43, and is further transported by the rack transport section 43, whereby the sample can be supplied to the measurement unit 2. In addition, the sample rack L accommodating the samples which are completely aspirated is moved to the after-analysis rack delivery position 461 by the rack transport section 43, and is delivered to the after-analysis rack holding section 42 by the rack delivery section 46. When the plural sample racks L are disposed on the before-analysis rack holding section 41, the sample racks L accommodating the samples which are completely analyzed are sequentially delivered to the after-analysis rack holding section 42 by the rack delivery section 46. These plural sample racks L are then stored in the after-analysis rack holding section 42.

### <Configuration of Information Processing Unit>

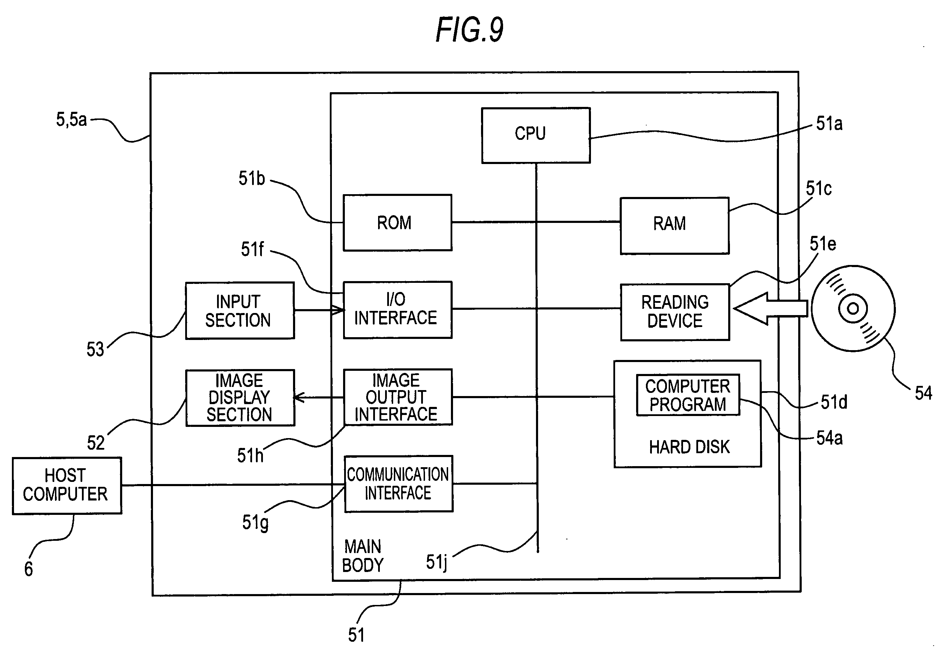

Next, the configuration of the information processing unit 5 will be described. The information processing unit 5 is composed of a computer. Fig. 9 is a block diagram illustrating the configuration of the information processing unit 5. The information processing unit 5 is realized by a computer 5a. As shown in Fig. 9, the computer 5a includes a main body 51, an image display section 52 and an input section 53. The main body 51 includes a CPU 51a, a ROM 51b, a RAM 51c, a hard disk 51d, a reading device 51e, an I/O interface 51f, a communication interface 51g, and an image output interface 51h. The CPU 51a, ROM 51b, RAM 51c, hard disk 51d, reading device 51e, I/O interface 51f, communication interface 51g, and image output interface 51h are connected to each other by a bus 51j.

The CPU 51a can execute a computer program loaded to the RAM 51c. The CPU 51a executes a computer program 54a for analyzing blood and for controlling the measurement unit 2 and the sample transport unit 4, which will be described later, so that the computer 5a functions as the information processing unit 5.

The ROM 51b is composed of a mask ROM, a PROM, an EPROM, an EEPROM or the like. The computer program executed by the CPU 51a, the data used for the computer program, and the like are recorded in the ROM 51b.

The RAM 51c is composed of a SRAM, a DRAM or the like. The RAM 51c is used to read the computer program 54a recorded in the hard disk 51d. In addition, the RAM 51c is used as an operating area of the CPU 51a when the CPU 51a executes a computer program.

In the hard disk 51d, various computer programs for execution by the CPU 51a, such as an operating system and an application program, and data which is used to execute the computer programs, are installed. The computer program 54a to be described later is also installed in the hard disk 51d. In addition, the computer program 54a is an event-driven computer program.

The reading device 51e is composed of a flexible disk drive, a CD-ROM drive, a DVD-ROM drive or the like. The reading device 51e can read the computer program or data recorded in a portable recording medium 54. In the portable recording medium 54, the computer program 54a for prompting the computer to function as the information processing unit 5 is stored. The computer 5a can read the computer program 54a from the portable recording medium 54 and install the computer program 54a in the hard disk 51d.

The computer program 54a is provided by the portable recording medium 54 and can also be provided from an external device, which is connected to the computer 5a by an electric communication line (which may be wired or wireless) to communicate therewith, through the electric communication line. For example, when the computer program 54a is stored in a hard disk of a server computer on the internet, the computer 5a can access the server computer to download the computer program and install the computer program in the hard disk 51d.

Furthermore, in the hard disk 51d, for example, a multitasking operating system such as Windows (registered trademark), which is made and distributed by Microsoft corporation in U.S.A, is installed. In the following description, the computer program 54a according to this embodiment operates on the above operating system.

The I/O interface 51f is composed of, for example, a serial interface such as USB, IEEE1394 or RS-232C, a parallel interface such as SCSI, IDE or IEEE1284, and an analog interface including a D/A converter and an A/D converter. The input section 53 composed of a keyboard and a mouse is connected to the I/O interface 51f. The user can use the input section 53 so as to input data to the computer 5a. In addition, the I/O interface 51f is connected to the measurement unit 2 and the sample transport unit 4. Therefore, the information processing unit 5 can control the measurement unit 2 and the sample transport unit 4, respectively.

The communication interface 51g is an Ethernet (registered trademark) interface. The communication interface 51g is connected to a host computer 6 via a LAN (see Fig. 4). Via the communication interface 51g, the computer 5a can send and receive data to and from the host computer 6 connected to the LAN by using a predetermined communication protocol.

The image output interface 51h is connected to the image display section 52 composed of an LCD or a CRT so as to output a picture signal corresponding to the image data provided from the CPU 51a to the image display section 52. The image display section 52 displays an image (screen) in accordance with an input picture signal.

### [Measurement Operation of Blood Analyzer 1]

Hereinafter, an operation of the blood analyzer 1 according to this embodiment will be described.

### <Sample Measurement Operation>

First, the sample measurement operation of the blood analyzer 1 according to this embodiment will be described. The blood analyzer 1 can perform the measurement of a lymphoblast/nucleated red blood cell group, and the measurement of NRBC (nucleated red blood cells), using an optical detector D. The measurement process is composed of a first measurement process for measuring an L-Blast assay sample, a second measurement process for measuring an NRBC assay sample, and a data processing process for analyzing and processing the measurement data obtained by the first measurement process and the second measurement process.

First, an operator places the sample rack L holding the sample containers T on the before-analysis rack holding section 41. The rack sending sections 41b contact the sample rack L placed on the before-analysis rack holding section 41, and are moved backward and then transported to the rack transport section 43. Thereafter, the sample rack L is transported by the rack transport section 43, and the sample container T where a sample to be measured is accommodated is positioned at the sample supply position 43a. Next, the sample container T is grasped by the hand section 25a of the measurement unit 2, and the sample container T is taken out from the sample rack L. The hand section 25a is then oscillated, so that the sample in the sample container T is stirred. Next, the sample container T is inserted into the sample container setting section 25b. Next, the sample container setting section 25b moves in the Y direction, the sample bar-code is read by the bar-code reading section 26, and then the sample container T arrives at the aspirating position. Thereafter, the following first measurement process and second measurement process are performed.

### First measurement process

First, the first measurement process will be described. The blood analyzer 1 prepares, in the first measurement process, an L-Blast assay sample by mixing a complete blood sample (19.0 µL) and a first reagent (1.02 mL). Then, the L-Blast assay sample is measured by an optical detector D in accordance with a flow cytometry method.

Here, the first reagent was used which is composed of the following components.

### <First Reagent>

| | |
|---|---|
| MOPSO | 2.25 g/L |
| Polyoxyethylene(20) oleyl ether | 10.0 g/L |
| Sodium N-lauroylsarcosinate | 0.5 g/L |
| Proxel GXL | 0.40 g/L |
| EDTA-2K | 0.50 g/L |
| Xylitol | 40.22 g/L |
| NK-321 | 1.0 mg/L |
| pH: | 7.0 |
| Osmotic pressure: | 300 mOsm/Kg |
| Electric conductivity: | 0.745 mS/cm |

Further, the following three samples were used as complete blood samples.

**[Table 1]**

| | Lymphoblasts (L-Blast) | Nucleated red blood cells (NRBC) |
|---|---|---|
| Blood sample A | O | X |
| Blood sample B | X | X |
| Blood sample C | X | O |

In Table 1, "O" represents that there are target blood cells (lymphoblasts or nucleated red blood cells), and "X" represents that there are no target blood cells.

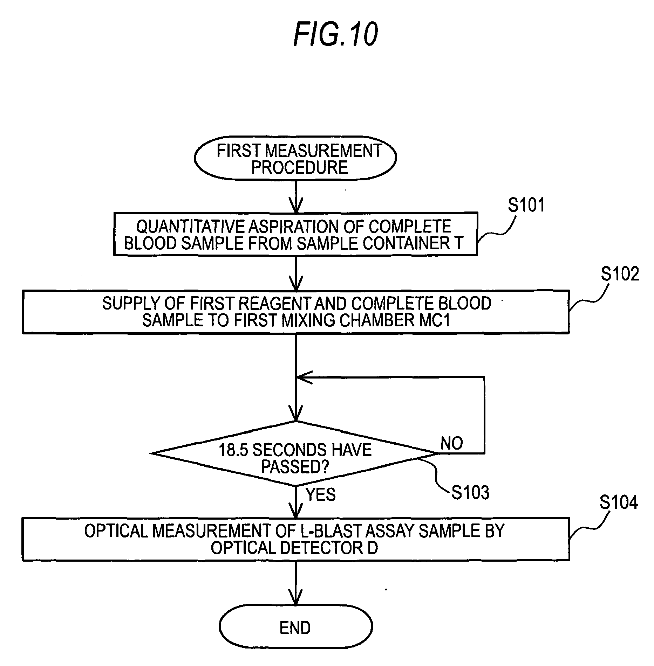

Fig. 10 is a flowchart illustrating the operation procedure of the blood analyzer 1 in the first measurement process. First, the CPU 51a controls the sample aspiration section 21, so that a given amount of the complete blood sample in the sample container T is aspired by the aspiration tube 211 (Step S101). Specifically, in processing of Step S101, the aspiration tube 211 is inserted into the sample container T, and a given amount of the complete blood sample (39.0 µL) is aspirated by the action of a syringe pump.

Next, the CPU 51a controls the measurement unit 2, whereby the first reagent (1.02 mL) from the reagent container 221 and the complete blood sample (19.0 µL) from the aspiration tube 211 are respectively supplied to the first mixing chamber MC1 (Step S102). The CPU 51a determines whether or not 18.5 seconds have passed after a supply of the first reagent and the complete blood sample to the first mixing chamber MC1 (Step S103), and waits for 18.5 seconds. Here, the first mixing chamber MC1 is warmed to 35.0°C by a heater. Accordingly, a mixture of the first reagent and the blood sample is warmed to 35.0°C for 18.5 seconds, and therefore the L-Blast assay sample is prepared.

Then, the L-Blast assay sample is subjected to optical measurement using an optical detector D (Step S104). Specifically, in processing of Step S104, the L-Blast assay sample and a sheath fluid are simultaneously supplied to a flow cell 231 of the optical detector D. At that time, forward-scattered light is received by the photodiode 243, and side-scattered light is received by the photodiode 246 and then the avalanche photodiode 248. Output signals (analog signals) being output by the respective light-receiving elements of the optical detector D are converted into digital signals by an A/D converter (not shown). And, a given signal processing is performed by a signal processing circuit (not shown), such that the digital signals are converted into digital data, i. e. first measurement data. The converted first measurement data is transmitted to the information processing unit 5. In this signal processing, a forward-scattered light signal (forward-scattered light intensity), a side-scattered light signal (side-scattered light intensity), and a side fluorescence signal (side fluorescence intensity) can be obtained as feature parameters contained in the first measurement data. In this way, the first measurement process is completed. In addition, as will be illustrated hereinafter, the CPU 51a of the information processing unit 5 performs a given analysis processing on the first measurement data. Accordingly, the analysis result data is generated including numerical data such as NEUT, LYMPH, EO, BASO, MONO and WBC, and the analysis result data is recorded in the hard disk 51d.

### Second measurement process

Next, the second measurement process will be described. The second measurement process is temporally overlapped with a part of the first measurement process. The blood analyzer 1, in the second measurement process, prepares an NRBC assay sample by mixing a complete blood sample (17.0 µL) with a second reagent (1.0 mL) and a third reagent (0.030 mL). The NRBC assay sample is measured in the optical detector D by a flow cytometry method. As the second reagent, the above-mentioned Stromatolyser NR hemolytic reagent was used. As the third reagent, the above-mentioned Stromatolyser NR dye solution was used.

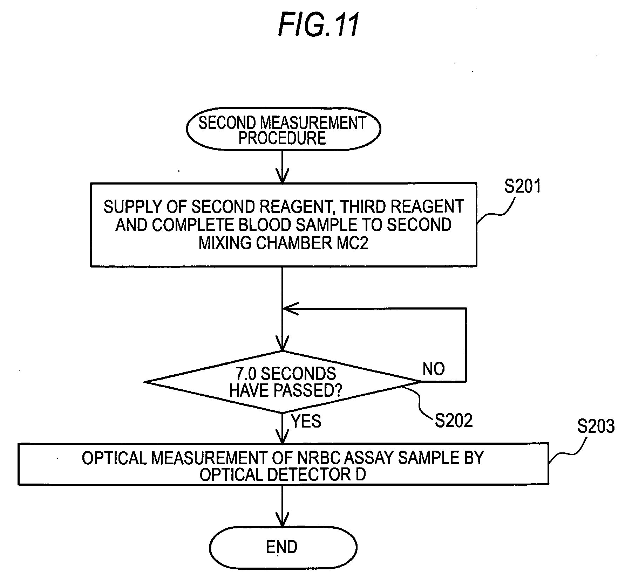

Fig. 11 is a flowchart illustrating the operation procedure of the blood analyzer 1 in the second measurement process. The CPU 51a controls the measurement unit 2, so that a second reagent (1.0 mL) from the reagent container 222a, a third reagent (0.030 mL) from the reagent container 222b, and a complete blood sample (17.0 µL) from the aspiration tube 211 are respectively supplied to the second mixing chamber MC2 (Step S201). In Step S201, the sample supplied to the second mixing chamber MC2 is a portion of the complete blood sample aspirated by the aspiration tube 211 in Step S101. That is, in Step S101, the sample supplied to the first mixing chamber MC1 and the sample supplied to the second mixing chamber MC2 are aspirated from the sample container T at one time.

Next, the CPU 51a determines whether or not 7.0 seconds have passed after a supply of the second reagent, the third reagent and the complete blood sample to the second mixing chamber MC2 (Step S202), and waits for 7. 0 seconds. Here, the second mixing chamber MC2 is warmed to 41.0°C by a heater. Accordingly, a mixture of the second reagent, the third reagent and the blood sample is warmed to 41.0°C for 7.0 seconds, and therefore the NRBC assay sample is prepared.

Then, the NRBC assay sample is subjected to optical measurement using an optical detector D (Step S203). Specifically, in processing of Step S203, the NRBC assay sample and the sheath fluid are simultaneously supplied to a flow cell 231 of the optical detector D. At that time, forward-scattered light is received by the photodiode 243, and side-scattered light is received by the photodiode 246 and then the avalanche photodiode 248. Output signals (analog signals) being output by the respective light-receiving elements of the optical detector D are converted into digital signals, in the same manner as in the first measurement process. A given signal processing is then performed such that the digital signals are converted into digital data, i.e. second measurement data. The converted second measurement data is transmitted to the information processing unit 5. In this signal processing, a forward-scattered light signal (forward-scattered light intensity), a side-scattered light signal (side-scattered light intensity), and a side fluorescence signal (side fluorescence intensity) can be obtained as feature parameters contained in the second measurement data. In this way, the second measurement process is completed. In addition, as will be illustrated hereinafter, the CPU 51a of the information processing unit 5 performs a given analysis processing on the second measurement data. Accordingly, the analysis result data is generated including numerical data of NRBC, and the analysis result data is recorded in the hard disk 51d.

### Data processing process

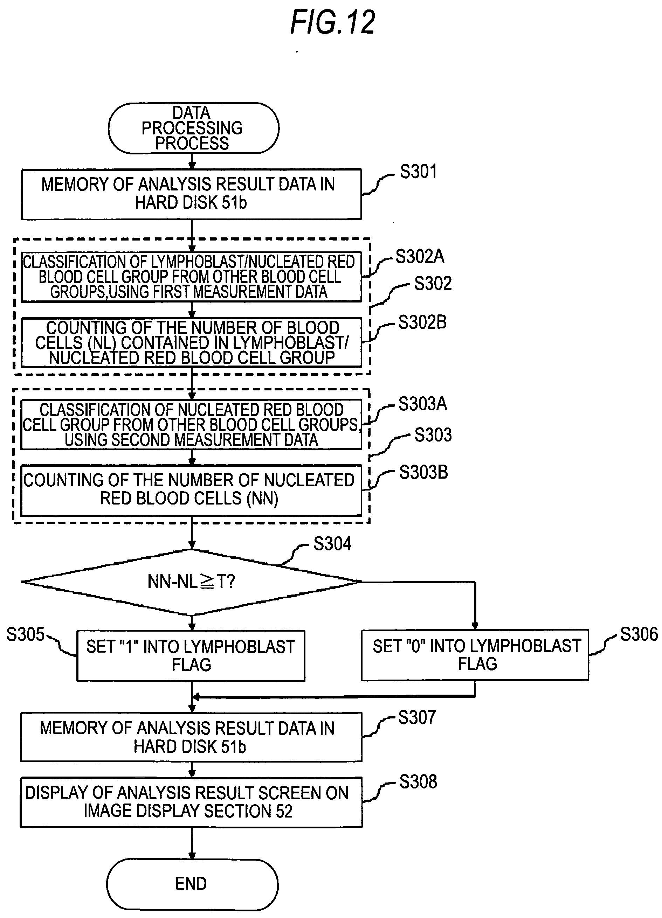

Next, a data processing process will be described. Fig. 12 is a flowchart illustrating the processing procedure of the blood analyzer 1 in the data processing process. The information processing unit 5 of the blood analyzer 1 receives measurement data from the measurement unit 2 (Step S301). The received measurement data contains the first measurement data and the second measurement data. The computer program 54a executed by the CPU 51a is an event-driven program. When an event receiving the measurement data takes place, a processing of Step S302 is called.

In Step S302, the CPU 51a performs the classification of a lymphoblast/nucleated red blood cell group from other blood cell groups using the first measurement data, and the counting of blood cells contained in the lymphoblast/nucleated red blood cell group (Step S302). This processing will be described in more detail.

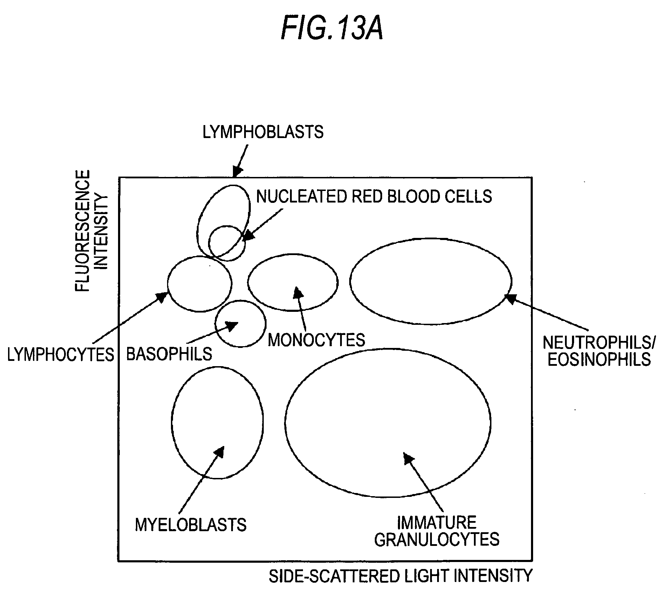

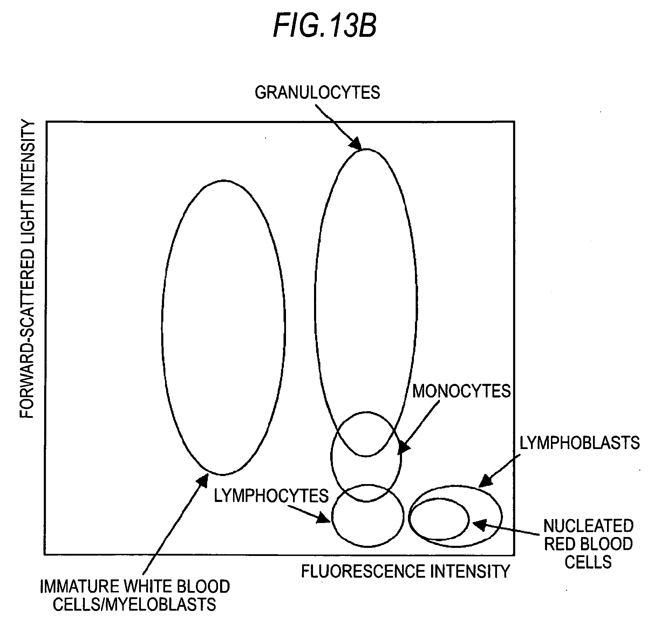

Fig. 13A is a scattergram (particle size distribution) of side-scattered light intensity and side fluorescence intensity in the first measurement data. Fig. 13B is a scattergram of forward-scattered light intensity and side fluorescence intensity in the first measurement data. On the scattergram of side-scattered light intensity and side fluorescence intensity in the first measurement data shown in Fig. 13A, there are appeared a cluster of myeloblasts, a cluster of immature granulocytes, a cluster of basophils, a cluster of a blood cell group composed of neutrophils and eosinophils, a cluster of lymphocytes, a cluster of monocytes, and a cluster of a lymphoblast/nucleated red blood cell group. In addition, on the scattergram of forward-scattered light intensity and side fluorescence intensity in the first measurement data shown in Fig. 13B, there are appeared a cluster of a blood cell group composed of immature granulocytes and myeloblasts, a cluster of granulocytes (a blood cell group composed of neutrophils, eosinophils and basophils), a cluster of monocytes, a cluster of lymphocytes, and a cluster of a lymphoblast/nucleated red blood cell group. As shown in these scattergrams, the use of the fluorescence intensity of the first measurement data can provide discrimination of the lymphoblast/nucleated red blood cell group cluster from other clusters. In processing of Step S302, the CPU 51a distinguishes the lymphoblast/nucleated red blood cell group from other clusters, using side-scattered light intensity and fluorescence intensity of the first measurement data, and therefore detects the lymphoblast/nucleated red blood cell group (Step S302A). And, the CPU 51a counts the number of blood cells contained in the detected lymphoblast/nucleated red blood cell group (Step S302B).

Next, in Step S303, the CPU 51a performs the classification of the nucleated red blood cell group from other blood cell groups, using the second measurement data, and the counting of nucleated red blood cells (Step S303). This processing will be described in more detail.

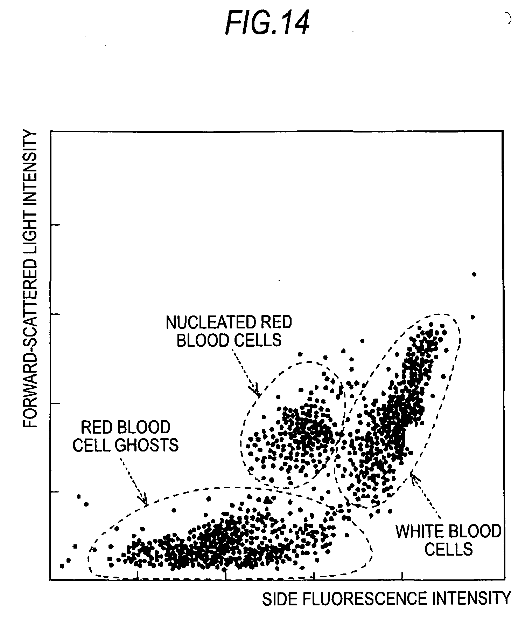

Fig. 14 is a scattergram of forward-scattered light intensity and side fluorescence intensity in the second measurement data. On the scattergram of forward-scattered light intensity and side fluorescence intensity in the second measurement data shown in Fig. 14, there are appeared a cluster of nucleated red blood cells, a cluster of white blood cells, and a cluster of red blood cell ghosts. As shown in this scattergram, the use of the forward-scattered light intensity and side fluorescence intensity of the second measurement data can provide discrimination of the nucleated red blood cell cluster from other clusters. In processing of Step S303, the CPU 51a discriminates the nucleated red blood cells from other clusters, using forward-scattered light intensity and fluorescence intensity of the second measurement data, and therefore detects the nucleated red blood cells (Step S303A). And, the CPU 51a counts the number of the detected nucleated red blood cells (Step S303B).

Next, in Step S304, the CPU 51a determines whether or not a difference between the number of blood cells (NL) contained in the lymphoblast/nucleated red blood cell group obtained in Step S302 and the number of nucleated red blood cells (NN) obtained in Step S303 is equal to or higher than a given base value T (Step S304). The base value T is a value such that when an NL-NN value is equal to or higher than the base value T, it can be determined that lymphoblasts are contained in the blood sample, and when an NL-NN value is less than the base value T, it can be determined that lymphoblasts are not contained in the blood sample. The base value T is previously set taking into consideration an error in the number of blood cells (NL) contained in the lymphoblast/nucleated red blood cell group and the number of nucleated red blood cells (NN). When NL-NN≥T in this processing ("YES" in Step S304), it can be determined that lymphoblasts are contained in the blood sample. Therefore, in this case, the CPU 51a sets "1" into a lymphoblast flag provided in the RAM 51c (Step S305). In this connection, the lymphoblast flag is a flag reflecting the existence and nonexistence of lymphoblasts in the blood sample. When "1" is set into the lymphoblast flag, this represents the presence of lymphoblasts. When "0" is set into the lymphoblast flag, this represents the absence of lymphoblasts. Thereafter, the CPU 51a switches to a processing of Step S307.

On the other hand, when NL-NN<T in Step S304 ("NO" in Step S304), it can be determined that lymphoblasts are not contained in the blood sample. Therefore, in this case, the CPU 51a sets "0" into the lymphoblast flag (Step S306). Thereafter, the CPU 51a switches to a processing of Step S307.

In Step S307, the CPU 51a stores the thus obtained analysis results (including the nucleated red blood cell count, and the lymphoblast flag) in the hard disk 51d (Step S307). Then, the CPU 51a displays an analysis result screen displaying the analysis results stored in the hard disk 51d on an image display section 52 (Step S308), and then terminates the processing.

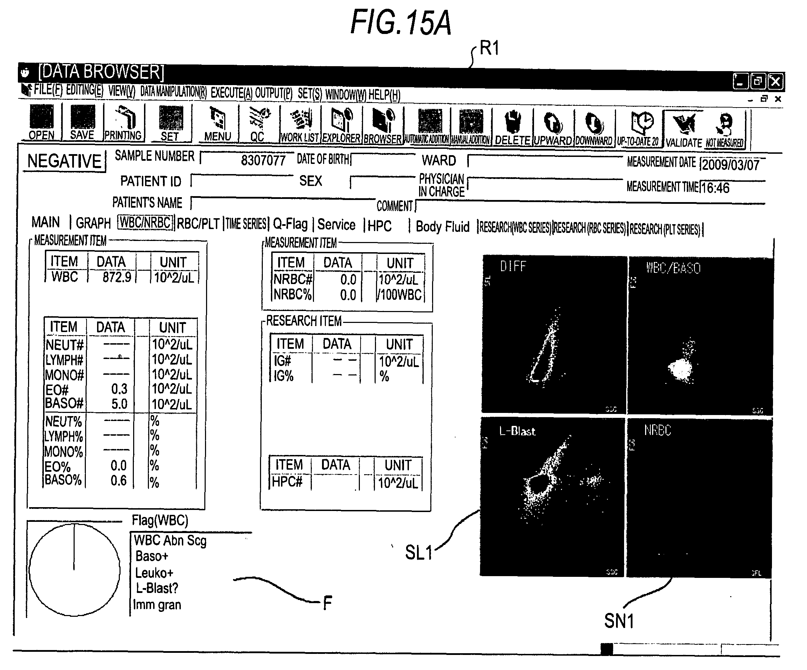

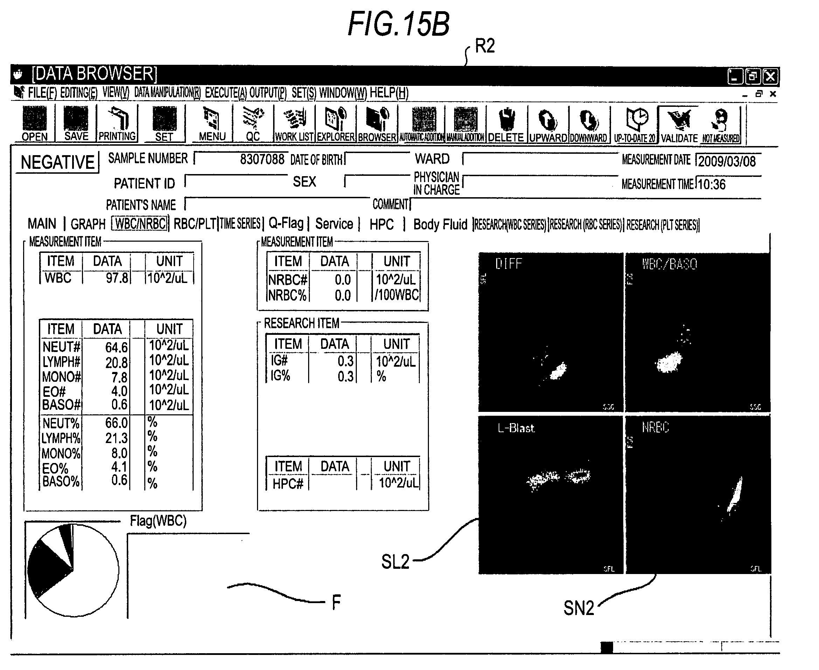

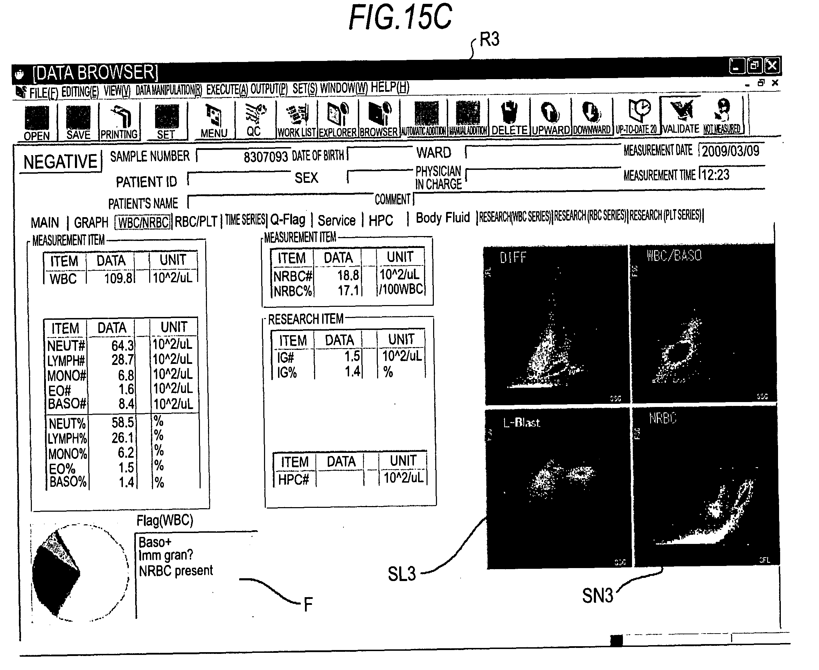

Figs. 15A, 15B and 15C are views illustrating an analysis result screen of the blood analyzer 1. Fig. 15A shows an analysis result screen of a blood sample A. Fig. 15B shows an analysis result screen of a blood sample B. Fig. 15C shows an analysis result screen of a blood sample C. As shown in Figs. 15A, 15B and 15C, numerical data of the measured measurement items (WBC, RBC, PLT, NRBC, etc.) is displayed on the analysis result screens R1, R2 and R3. Lymphoblasts are contained in the blood sample A. For this reason, in the analysis result data relating to the blood sample A, the lymphoblast flag is set with "1". Therefore, on the analysis result screen R1 of the blood sample A, a column F of Flag is attached with an indication "L-Blast?" which is information representing a possibility of the presence of a lymphoblast item, as shown in Fig. 15A. On the other hand, lymphoblasts are not contained in the blood sample B and blood sample C. For this reason, in the analysis result data relating to the blood sample B and the analysis result data relating to the blood sample C, the lymphoblast flag is set with "0". Therefore, on the analysis result screen R2 of the blood sample B and the analysis result screen R3 of the blood sample C, an indication "L-Blast?" is not attached, as shown in a column F of Flag of Figs. 15B and 15C. The blood sample C contains nucleated red blood cells. Therefore, on the analysis result screen R3 of the blood sample C, a column F of Flag is attached with an indication "NRBC present" which is information representing the presence of nucleated red blood cells, as shown in Fig. 15C. Accordingly, the operator can grasp whether or not lymphoblasts were detected from the blood sample, even only by watching the analysis result screen. In addition, on the analysis result screens R1, R2 and R3 are displayed scattergrams SL1, SL2 and SL3 of side-scattered light intensity and side fluorescence intensity of the first measurement data. On the analysis result screens R1, R2 and R3 are displayed scattergrams SN1, SN2 and SN3 of side-scattered light intensity and side fluorescence intensity of the second measurement data. By referring to these scattergrams, the operator can grasp grounds of the detection results for the existence and nonexistence of lymphoblasts by the blood analyzer 1. Further, the operator can determine the validity of the detection results for the existence and nonexistence of lymphoblasts by the blood analyzer 1.

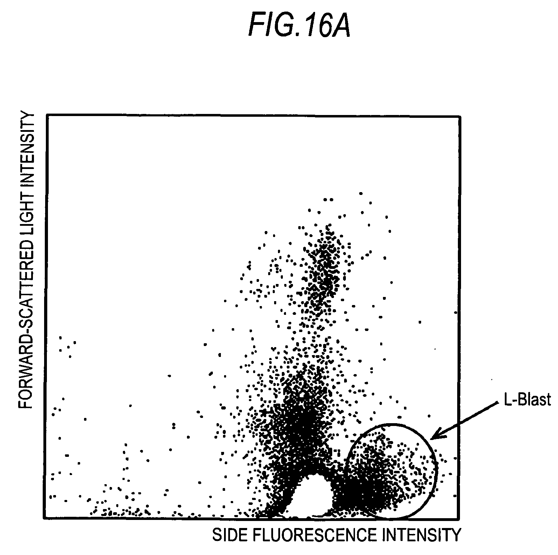

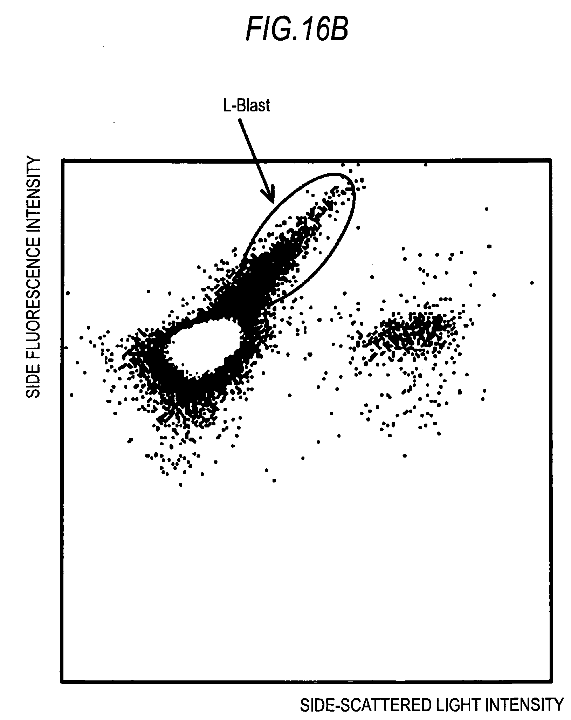

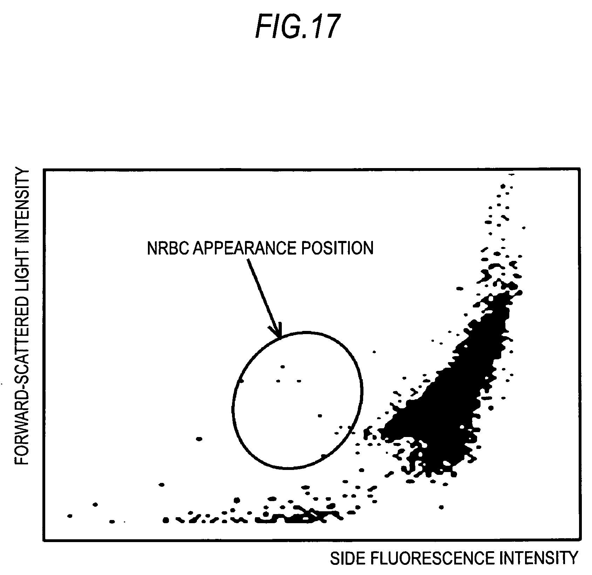

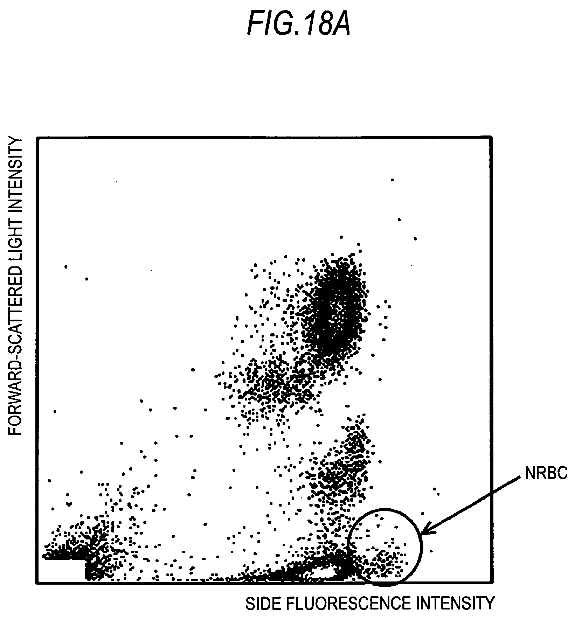

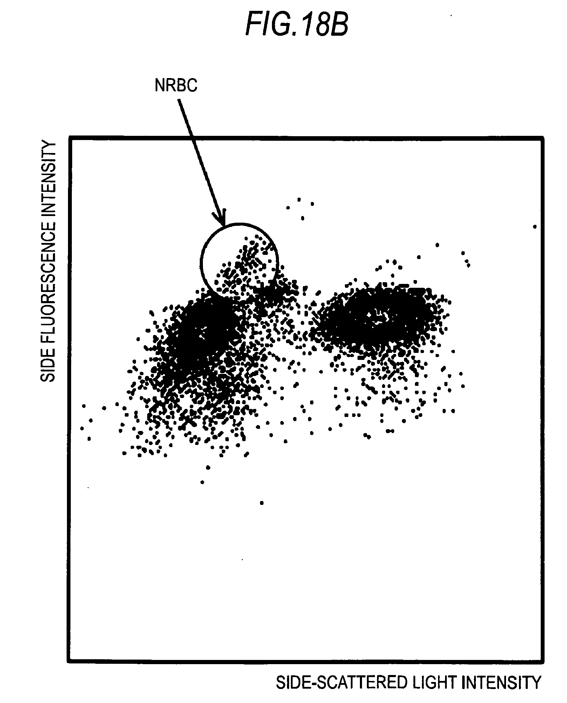

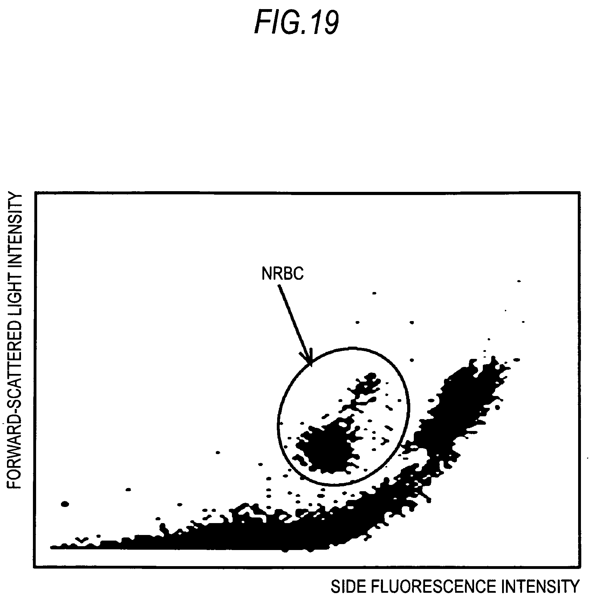

By using a specific example of the scattergram, the detection of lymphoblasts will be described in more detail. Fig. 16A is a scattergram of forward-scattered light intensity and fluorescence intensity in the first measurement data of the blood sample A. Fig. 16B is a scattergram of side-scattered light intensity and fluorescence intensity in the first measurement data of the blood sample A. Fig. 17 is a scattergram of forward-scattered light intensity and fluorescence intensity in the second measurement data of the blood sample A. Fig. 18A is a scattergram of forward-scattered light intensity and fluorescence intensity in the first measurement data of the blood sample C. Fig. 18B is a scattergram of side-scattered light intensity and fluorescence intensity in the first measurement data of a blood sample C. Fig. 19 is a scattergram of forward-scattered light intensity and fluorescence intensity in the second measurement data of the blood sample C.

As can be seen from Figs. 16A and 16B, in the blood sample A, a cluster of the lymphoblast/nucleated red blood cell group can be confirmed on the scattergram. On the other hand, as can be seen from Fig. 17, in the blood sample A, a cluster of nucleated red blood cells cannot be confirmed on the scattergram. These results represent that a cluster of the lymphoblast/nucleated red blood cell group appearing in Figs. 16A and 16B is formed mainly of lymphoblasts. Therefore, it can be seen that there are lymphoblasts in the blood sample A.

As can be seen from Figs. 18A and 18B, in the blood sample C, a cluster of the lymphoblast/nucleated red blood cell group can be confirmed on the scattergram. In addition, a cluster of the lymphoblast/nucleated red blood cell group in the blood sample C is smaller in scale than a cluster of the lymphoblast/nucleated red blood cell group in the blood sample A, thus representing that the blood sample C has a lower number of blood cells. As can be seen from Fig. 19, in the blood sample C, a cluster of nucleated red blood cells can be confirmed on the scattergram. These results represent that a cluster of the lymphoblast/nucleated red blood cell group appearing in Figs. 18A and 18B is formed mainly of nucleated red blood cells. That is, it is suggested that there are nucleated red blood cells, not lymphoblasts, in the blood sample C.

In this manner, by referring to the scattergrams that can be obtained from the first measurement data and the second measurement data, grounds of the detection results for the existence and nonexistence of lymphoblasts by the blood analyzer 1 can be more accurately grasped. Further, the operator can determine the validity of the detection results for the existence and nonexistence of lymphoblasts by the blood analyzer 1.

According to the configuration as described above, the blood analyzer 1 can detect the lymphoblast/nucleated red blood cell group through the measurement of an L-Blast assay sample prepared by mixing a blood sample with a first reagent containing a nucleic acid-staining fluorescent dye by means of an optical detector D, and can measure the number of blood cells contained in the lymphoblast/nucleated red blood cell group. Further, based on the number of blood cells and the number of nucleated red blood cells obtained by the second measurement process, it is possible to detect whether or not lymphoblasts are contained in the blood sample. According to the above-mentioned measurement of the blood analyzer 1, it is possible to detect lymphoblasts without the use of a fluorescence-labeled antibody. As a consequence, it is possible to detect lymphoblasts while reducing measurement costs.

### (Other Embodiments)

In the sample preparation section 22, there is no particular limit to the reaction temperature and the reaction time, upon mixing of the blood sample and the first reagent. Therefore, the reaction temperature and time may be appropriately established depending on the damaged or stained state of blood cells in the blood sample. Specifically, if the reaction temperature is high, the reaction time may be shortened. If the reaction temperature is low, the reaction time may be adjusted to be longer. More specifically, mixing of the blood sample and the reagent is preferably performed at a temperature of 20°C to 40°C for 3 to 20 seconds.

In the above-mentioned embodiment, even though there has been described the configuration in which the first measurement process is performed using a first reagent containing a hemolytic agent and a nucleic acid-staining fluorescent dye, the present invention is not limited thereto. Alternatively, the first measurement process may also be configured to include separately preparing a reagent containing a hemolytic agent and a reagent containing a nucleic acid-staining dye, mixing these two reagents with a blood sample to prepare an L-Blast assay sample, and detecting a lymphoblast/nucleated red blood cell group and counting the number of blood cells in the lymphoblast/nucleated red blood cell group. In this case, concentrations of a surfactant, a solubilizing agent and a fluorescent dye are adjusted to the above-specified concentration range when the above-mentioned two reagents were mixed. Here, a mixing ratio of the hemolytic agent-containing reagent and the nucleic acid-staining dye-containing reagent is preferably in the range of 1000:1 to 10:1.

Even though there is no particular limit to the order of mixing individual reagents of the reagent kit with the blood sample when it is desired to use the above-mentioned reagent kit, it is preferred that two reagents are mixed, and then the blood sample is mixed to the mixed reagents.

In the above-mentioned embodiment, even though there has been described the configuration which includes performing the first measurement process and the second measurement process, and detecting whether or not lymphoblasts are contained in the blood sample, using first measurement data that can be obtained by the first measurement process and second measurement data that can be obtained by the second measurement process, the present invention is not limited thereto. As shown in Fig. 13A, in a cluster of the lymphoblast/nucleated red blood cell group, a lower fluorescence intensity part shows an overlap between the lymphoblast cluster and the nucleated red blood cell cluster, whereas a higher fluorescence intensity part shows an appearance of lymphoblasts only. For this reason, in the cluster of the lymphoblast/nucleated red blood cell group, a base value of fluorescence intensity is provided near an upper limit of the fluorescence intensity of the nucleated red blood cell cluster. When particles having a fluorescence intensity equal to or higher than the base value are detected in the cluster of the lymphoblast/nucleated red blood cell group, it can be determined that there are lymphoblasts in the blood sample. When particles having a fluorescence intensity equal to or higher than the base value are not detected in the cluster of the lymphoblast/nucleated red blood cell group, it can be determined that there are no lymphoblasts in the blood sample. In this case, it is possible to determine the existence and nonexistence of lymphoblasts even without performing the second measurement process for the detection of nucleated red blood cells. Therefore, the configuration of the blood analyzer 1 can be further simplified and measurement costs can also be reduced.

In the above-mentioned embodiment, even though there has been described the configuration which includes detecting a lymphoblast/nucleated red blood cell group and counting blood cells contained in the lymphoblast/nucleated red blood cell group, based on the first measurement data, detecting nucleated red blood cells and counting nucleated red blood cells, based on the second measurement data, calculating a difference between the number of blood cells in the lymphoblast/nucleated red blood cell group and the number of nucleated red blood cells, and comparing the calculated difference and the base value T to determine the existence and nonexistence of lymphoblasts, the present invention is not limited thereto. For example, there may be a configuration in which the detection of the lymphoblast/nucleated red blood cell group is performed based on the first measurement data, and the detection of nucleated red blood cells is performed based on the second measurement data, it is determined that lymphoblasts are present in the blood sample if there are particles being detected as the lymphoblast/nucleated red blood cell group and there are no particles being detected as nucleated red blood cells, and it is determined that there are no lymphoblasts in the blood sample for other cases than the above-mentioned cases. Further, a difference between the number of blood cells in the lymphoblast/nucleated red blood cell group and the number of nucleated red blood cells, in terms of the number of lymphoblasts, can be displayed on the analysis result screen. Further, with regard to the difference between the number of blood cells in the lymphoblast/nucleated red blood cell group and the number of nucleated red blood cells, the resulting numerical value obtained from deduction of a given numerical value from the calculated difference, in terms of the number of lymphoblasts, can be displayed on the analysis result screen.

In the above-mentioned embodiment, there has been described the configuration in which if a difference (NL-NN value) between the number of blood cells (NL) contained in the lymphoblast/nucleated red blood cell group and the number of nucleated red blood cells (NN) is equal to or higher than a base value T, it is determined that there are lymphoblasts in the blood sample, and if an NL-NN value is less than the base value T, it is determined that there are no lymphoblasts in the blood sample. However, the present invention is not limited thereto. For example, there may be a configuration in which if the number of blood cells (NL) contained in the lymphoblast/nucleated red blood cell group is larger than the number of nucleated red blood cells (NN), it is determined that there are lymphoblasts in the blood sample, and if the number of blood cells (NL) is equal to or less than the number of nucleated red blood cells (NN), it is determined that there are no lymphoblasts in the blood sample.

As described above, on the scattergram of side-scattered light intensity and side fluorescence intensity in the first measurement data shown in Fig. 13A, there appear a cluster of myeloblasts, a cluster of immature granulocytes, a cluster of basophils, a cluster of a blood cell group composed of neutrophils and eosinophils, a cluster of lymphocytes, a cluster of monocytes, and a cluster of a lymphoblast/nucleated red blood cell group. Therefore, there may also be a configuration in which the myeloblast cluster is classified from other clusters, using the side-scattered light intensity and side fluorescence intensity in the first measurement data, and when there are blood cells contained in the myeloblast cluster, the information representing the presence thereof or the number of blood cells (myeloblast count) contained in the myeloblast cluster is displayed on the analysis result screen. Further, there may also be a configuration in which the immature granulocyte cluster is classified from other clusters, using the side-scattered light intensity and side fluorescence intensity in the first measurement data, and when there are blood cells contained in the immature granulocyte cluster, the information representing the presence thereof or the number of blood cells (immature granulocyte count) contained in the immature granulocyte cluster is displayed on the analysis result screen.

In addition, there may also be a configuration in which mature white blood cells are classified into lymphocytes, basophils, monocytes, and a blood cell group composed of neutrophils and eosinophils, using side-scattered light intensity and side fluorescence intensity in the first measurement data, individual blood cells are counted for lymphocytes, basophils, monocytes, and blood cells contained in the blood cell group composed of neutrophils and eosinophils, and the number of individual blood cells is displayed on the analysis result screen. Further, there may also be a configuration in which, assuming that basophils are also contained in the blood cell group composed of neutrophils and eosinophils, mature white blood cells are classified into lymphocytes, monocytes and granulocytes, distinctively from lymphocytes and monocytes as granulocytes, blood cells contained in each cluster are counted, and the number of lymphocytes, monocytes, and granulocytes is displayed on the analysis result screen.

In the above-mentioned embodiment, even though there has been described the configuration which includes detecting the lymphoblast/nucleated red blood cell group, using side-scattered light intensity and side fluorescence intensity in the first measurement data, the present invention is not limited thereto. As shown in Fig. 13B, on the scattergram of forward-scattered light intensity and side fluorescence intensity in the first measurement data, there appear a cluster of a blood cell group composed of immature granulocytes and myeloblast, a cluster of granulocytes (blood cell group composed of neutrophils, eosinophils and basophils), a cluster of monocytes, a cluster of lymphocytes, and a cluster of a lymphoblast/nucleated red blood cell group. Therefore, there may also be a configuration in which a lymphoblast/nucleated red blood cell group is detected using forward-scattered light intensity and side fluorescence intensity in the first measurement data, and then blood cells contained in the lymphoblast/nucleated red blood cell group are counted. Further, there may also be a configuration in which a cluster of the blood cell group composed of myeloblasts and immature granulocytes is classified from other clusters, using forward-scattered light intensity and side fluorescence intensity in the first measurement data, and when there are blood cells contained in a cluster of the blood cell group composed of myeloblasts and immature granulocytes, the information representing the presence thereof or the number of blood cells contained in a cluster of the blood cell group composed of myeloblasts and immature granulocytes is displayed on the analysis result screen.

Further, there may be a configuration which includes classification of mature white blood cells into lymphocytes, monocytes and granulocytes, using forward-scattered light intensity and side fluorescence intensity in the first measurement data, counting of lymphocytes, monocytes, and granulocytes, and display of the number of individual blood cells on the analysis result screen.

Further, as shown in Figs. 13A and 13B, it is possible to discriminate between a lymphoblast/nucleated red blood cell group, mature white blood cells (lymphocytes, monocytes, and granulocytes), and a blood cell group composed of myeloblasts and immature granulocytes, using only the side fluorescence intensity contained in the first measurement data. Therefore, there may be a configuration which includes classifying a region of the lymphoblast/nucleated red blood cell group (a high fluorescence intensity region), a region of mature white blood cells (a moderate fluorescence intensity region), and a region of the blood cell group composed of myeloblasts and immature granulocytes (a low fluorescence intensity region), using side fluorescence intensity contained in the first measurement data, counting the number of blood cells for each region, and calculating the number of blood cells in the lymphoblast/nucleated red blood cell group, the number of mature white blood cells, and the number of blood cells in the blood cell group composed of myeloblasts and immature granulocytes.

In the above-mentioned embodiment, even though there has been described the configuration in which the second measurement process is performed by the blood analyzer 1, and the nucleated red blood cell group is detected in the data processing process, the present invention is not limited thereto. That is, there may also be a configuration in which the information on the detection of the nucleated red blood cell group is obtained by other blood analyzers different from the blood analyzer 1 or by manual manipulations, and the thus obtained information is input using an input device of the blood analyzer 1. Here, as the input device of the blood analyzer 1, mention may be made of the above-exemplified input section 53 and communication interface 51g. More specifically, the information on the detection of the nucleated red blood cell group can be input to the blood analyzer 1, using the input section 53 composed of a keyboard and a mouse, which is connected to the I/O interface 51f. In addition, the blood analyzer 1 can be connected to the above-mentioned other blood analyzers using the communication interface 51g, and the information on the detection of the nucleated red blood cell group can be input to the blood analyzer 1 through the medium of the communication interface 51g.

In the above-mentioned embodiment, even though there has been described the configuration in which controlling of the measurement unit 2 and processing of the measurement data are performed through the execution of the computer program 54a by the CPU 51a, the present invention is not limited thereto. There may also be a configuration in which controlling of the measurement unit 2 and processing of the measurement data are performed by using special hardware, such as FPGA or ASIC, which can perform the same processes carried out by the computer program 54a.

Further, in the above-mentioned embodiment, the configuration has been described such that all the processes of the computer program 54a are performed by the single computer 5a, but the invention is not limited thereto. The same processes carried out by the above-mentioned computer program 54a may be implemented by a distributed system in which the processes are distributed on and performed by a plurality of apparatuses (computers).

As discussed above, the sample examination system of the present invention is useful as a blood analyzer which performs the optical measurement of a blood sample, and the classification of cell groups contained in the blood sample into plural populations.

## Claims

1. A blood analyzer (1), comprising:
a blood sample supply section (43a) for supplying a first blood sample and a second blood sample divided from a blood sample;
a sample preparation section (22) for preparing a first assay sample by mixing the first blood sample, a first hemolytic agent and a first nucleic acid-staining fluorescent dye, and a second assay sample by mixing the second blood sample, a second hemolytic agent different from the first hemolytic agent and a second nucleic acid-staining fluorescent dye, wherein the first hemolytic agent and the first nucleic acid-staining fluorescent dye are contained in a first reagent, said first reagent is a reagent for detecting a blood cell group composed of lymphoblasts and nucleated red blood cells, and the second hemolytic agent is a hemolytic agent for the measurement of nucleated red blood cells;
a flow cell (231) for flowing the first assay sample and the second assay sample;
a light source (237) for irradiating a flow of the first assay sample or the second assay sample in the flow cell (231);
an optical detecting section (243, 246, 248) for receiving first fluorescence and first scattered light selected from forward-scattered light and side-scattered light emitted from the irradiated first assay sample, and receiving second fluorescence and second forward-scattered light from the irradiated second assay sample; and
a controller (5) configured to perform data processing operations comprising:
discriminating a cell group composed of lymphoblasts and nucleated red blood cells from other blood cells, contained in the first assay sample, on the basis of the first fluorescence and the first scattered light,
counting the discriminated cells contained in the cell group, discriminating nucleated red blood cells from other blood cells, contained in the second assay sample, on the basis of the second fluorescence and the second forward-scattered light,
counting the discriminated nucleated red blood cells, obtaining an information on the presence of the lymphoblasts in the blood sample, on the basis of the number of the cells in the cell group and the number of the nucleated red blood cells, and
outputting the information on the presence of the lymphoblasts in the blood sample.

2. The blood analyzer (1) according to claim 1,
wherein
the controller (5) further performs an operation of comparing
the number of cells contained in the cell group and the number of nucleated red blood cells, and
the obtaining operation is performed by obtaining the information on the presence of lymphoblasts on the basis of the comparative results.

3. The blood analyzer (1) according to claim 1,
wherein
the controller (5) further performs an operation of comparing a given value with a difference value between the number of cells contained in the cell group and the number of nucleated red blood cells, and
the obtaining operation is performed by obtaining the information on the presence of lymphoblasts on the basis of the comparative results.

4. The blood analyzer (1) according to any one of claim 1 to 3, wherein the information on the presence of lymphoblasts contains the number of lymphoblasts.

5. A method for determining the existence and nonexistence of lymphoblasts in a blood sample, the method comprising:
supplying a first blood sample and a second blood sample divided from the blood sample;
preparing a first assay sample by mixing the first blood sample with a first hemolytic agent and a first nucleic acid-staining fluorescent dye, wherein the first hemolytic agent and the first nucleic acid-staining fluorescent dye are contained in a first reagent, and the first reagent is a reagent for detecting a blood cell group composed of lymphoblast and nucleated red blood cells;
preparing a second assay sample by mixing the second blood sample with a second hemolytic agent different from the first hemolytic agent and a second nucleic acid-staining fluorescent dye, wherein the second hemolytic agent is a hemolytic agent for the measurement of nucleated red blood cells;
performing optical measurement of the first assay sample to obtain first output signals of first fluorescent light and first scattered light selected from forward-scattered light and side-scattered light;
performing optical measurement of the second assay sample to obtain second output signals of second fluorescent light and second forward-scattered light;
converting the first output signals and the second output signals to first measurement data and second measurement data, respectively;
discriminating a cell group composed of lymphoblasts and nucleated red blood cells from other blood cell groups based on the first measurement data;
counting the number of blood cells (NL) contained in said discriminated cell group;
discriminating nucleated red blood cells from other blood cell groups based on the second measurement data;
counting the number of nucleated red blood cells (NN) contained in said discriminated cell group; and
determining whether or not a difference between the number of the blood cells (NL) and the mummer of the nucleated red blood cells (NN) is equal to or higher than a base value.

6. A computer program (54a) embodied on a computer readable medium (54) for determining the existence and nonexistence of lymphoblasts in a blood sample, comprising:
a code segment for discriminating a cell group composed of lymphoblasts and nucleated red blood cells from other blood cell groups based on first measurement data, wherein said first measurement data are converted from first output signals of first fluorescent light and first scattered light selected from forward-scattered light and side-scattered light obtained by optical measurement of a first assay sample, the first assay sample being prepared by mixing a first blood sample divided from the blood sample with a first hemolytic agent and a first nucleic acid-staining fluorescent dye, wherein the first hemolytic agent and the first nucleic acid-staining fluorescent dye are contained in a first reagent, and the first reagent is a reagent for detecting a blood cell group composed of lymphoblast and nucleated red blood cells;
a code segment for counting the number of blood cells (NL) contained in said discriminated cell group;
a code segment for discriminating nucleated red blood cells from other blood cell groups based on second measurement data, wherein said second measurement data are converted from second output signals of second fluorescent light and second forward-scattered light obtained by optical measurement of a second assay sample, the second assay sample being prepared by mixing a second blood sample divided from the blood sample with a second hemolytic agent different from the first hemolytic agent and a second nucleic acid-staining fluorescent dye, wherein the second hemolytic agent is a hemolytic agent for the measurement of nucleated red blood cells;
a code segment for counting the number of nucleated red blood cells (NN) contained in said discriminated cell group; and
a code segment for comparing the number of the blood cells (NL) and the number of the nucleated red blood cells (NN).

## Patentansprüche

1. Blutanalysator (1), umfassend:
einen Blutprobenzufuhrbereich (43a) zum Zuführen einer von einer Blutprobe abgezweigten ersten und zweiten Blutprobe;
einen Probenvorbereitungsbereich (22) zum Vorbereiten einer ersten Assay-Probe durch Mischen der ersten Blutprobe, eines ersten hämolytischen Mittels und eines ersten Nukleinsäure-färbenden Fluoreszenzfarbstoffs und einer zweiten Assay-Probe durch Mischen der zweiten Probe, eines von dem ersten hämolytischen Mittel unterschiedlichen zweiten hämolytischen Mittels, und eines zweiten Nukleinsäure-färbenden Fluoreszenzfarbstoffs, wobei das erste hämolytische Mittel und der erste Nukleinsäure-färbende Fluoreszenzfarbstoff in einem ersten Reagens enthalten sind, wobei das erste Reagens ein Reagens zum Detektieren einer Blutzellgruppe aus Lymphoblasten und kernhaltigen roten Blutzellen ist und das zweite hämolytische Mittel ein hämolytisches Mittel zur Messung kernhaltiger roter Blutzellen ist;
eine Durchflusszelle (231) für den Durchfluss der ersten Assay-Probe und der zweiten Assay-Probe;
eine Lichtquelle (237) zum Bestrahlen eines Durchflusses der ersten Assay-Probe oder der zweiten Assay-Probe in der Durchflusszelle (231);
einen optischen Detektierbereich (243, 246, 248) zum Empfangen von erster Fluoreszenz und erstem gestreuten Licht, ausgewählt aus vorwärts-gestreutem Licht und seitwärts-gestreutem Licht, emittiert aus der bestrahlten ersten Assay-Probe, und Empfangen von zweiter Fluoreszenz und zweitem vorwärts-gestreutem Licht aus der bestrahlten zweiten Assay-Probe; und
einen Controller (5), konfiguriert zum Ausführen von Datenverarbeitungsoperationen, umfassend:
Diskriminieren einer Zellgruppe aus Lymphoblasten und kernhaltigen roten Blutzellen von anderen Blutzellen, die in der ersten Assay-Probe enthalten sind, auf der Basis der ersten Fluoreszenz und des ersten gestreuten Lichts,
Zählen der diskriminierten Zellen, die in der Zellgruppe enthalten sind,
Diskriminieren von kernhaltigen roten Blutzellen von anderen Blutzellen, die in der zweiten Assay-Probe enthalten sind, auf der Basis der zweiten Fluoreszenz und des zweiten vorwärts-gestreuten Lichts,
Zählen der diskriminierten kernhaltigen roten Blutzellen,
Erhalten einer Information über die Anwesenheit der Lymphoblasten in der Blutprobe, auf der Basis der Anzahl der Zellen in der Zellgruppe und der Anzahl der kernhaltigen roten Blutzellen, und
Ausgeben der Information über die Anwesenheit der Lymphoblasten in der Blutprobe.

2. Blutanalysator (1) gemäß Anspruch 1,
wobei
der Controller (5) ferner eine Operation des Vergleichens der Anzahl von Zellen, die in der Zellgruppe enthalten sind, und der Anzahl von kernhaltigen roten Blutzellen ausführt, und
die Operation des Erhaltens durch Erhalten der Information über die Anwesenheit von Lymphoblasten auf der Basis der Vergleichsergebnisse durchgeführt wird.

3. Blutanalysator (1) gemäß Anspruch 1,
wobei
der Controller (5) ferner eine Operation des Vergleichens eines gegebenen Werts mit einem Differenzwert zwischen der Anzahl von Zellen, die in der Zellgruppe enthalten sind, und der Anzahl der kernhaltigen roten Blutzellen ausführt, und
die Operation des Erhaltens durch Erhalten der Information über die Anwesenheit von Lymphoblasten auf der Basis der Vergleichsergebnisse ausgeführt wird.

4. Blutanalysator (1) gemäß einem der Ansprüche 1 bis 3,
wobei die Information über die Anwesenheit von Lymphoblasten die Anzahl von Lymphoblasten enthält.

5. Verfahren zum Bestimmen der Existenz oder Nichtexistenz von Lymphoblasten in einer Blutprobe, wobei das Verfahren umfasst:
Zuführen einer ersten Blutprobe und einer von der Blutprobe abgezweigten zweiten Blutprobe;
Vorbereiten einer ersten Assay-Probe durch Mischen der ersten Blutprobe mit einem ersten hämolytischen Mittel und einem ersten Nukleinsäure-färbenden Fluoreszenzfarbstoff, wobei das erste hämolytische Mittel und der erste Nukleinsäure-färbende Fluoreszenzfarbstoff in einem ersten Reagens enthalten sind und das erste Reagens ein Reagens zum Detektieren einer Blutzellgruppe aus Lymphoblasten und kernhaltigen Blutzellen ist;
Vorbereiten einer zweiten Assay-Probe durch Mischen der zweiten Blutprobe mit einem von dem ersten hämolytischen Mittel unterschiedlichen zweiten hämolytischen Mittel und einem zweiten Nukleinsäure-färbenden Fluoreszenzfarbstoff, wobei das zweite hämolytische Mittel ein hämolytisches Mittel zur Messung von kernhaltigen roten Blutzellen ist;
Ausführen optischer Messung an der ersten Assay-Probe, so dass erste Ausgabe-Signale von erstem fluoreszentem Licht und erstem gestreutem Licht, ausgewählt aus vorwärts-gestreutem Licht und seitwärts-gestreutem Licht, erhalten werden;
Ausführen optischer Messung an der zweiten Assay-Probe, so dass zweite Ausgabe-Signale von zweitem fluoreszenten Licht und zweitem vorwärts-gestreuten Licht erhalten werden;
Umwandeln der ersten Ausgabe-Signale und der zweiten Ausgabe-Signale in erste Messdaten, beziehungsweise zweite Messdaten;
Diskriminieren einer Zellgruppe aus Lymphoblasten und kernhaltigen roten Blutzellen von anderen Blutzellgruppen, basierend auf den ersten Messdaten;
Zählen der Anzahl von Blutzellen (NL), die in der diskriminierten Zellgruppe enthalten sind;
Diskriminieren kernhaltiger roter Blutzellen von anderen Blutzellgruppen, basierend auf den zweiten Messdaten;
Zählen der Anzahl von kernhaltigen roten Blutzellen (NN), die in der diskriminierten Zellgruppe enthalten sind; und
Bestimmen, ob eine Differenz zwischen der Anzahl der Blutzellen (NL) und der Anzahl der kernhaltigen roten Blutzellen (NN) gleich oder höher als ein Basiswert ist oder nicht.

6. Computerprogramm (54a) auf einem computerlesbaren Medium (54), zum Bestimmen der Existenz und Nichtexistenz von Lymphoblasten in einer Blutprobe, umfassend:
ein Codesegment zum Diskriminieren einer Zellgruppe aus Lymphoblasten und kernhaltigen roten Blutzellen von anderen Blutzellgruppen, basierend auf ersten Messdaten, wobei die ersten Messdaten umgewandelt sind aus ersten Ausgabe-Signalen von erstem Fluoreszenzlicht und erstem gestreuten Licht, ausgewählt aus vorwärts-gestreutem Licht und seitwärts-gestreutem Licht, erhalten durch optische Messung an einer ersten Assay-Probe, wobei die erste Assay-Probe durch Mischen einer von der Blutprobe abgezweigten ersten Blutprobe mit einem ersten hämolytischen Mittel und einem ersten Nukleinsäure-färbenden Fluoreszentfarbstoff vorbereitet wird, wobei das erste hämolytische Mittel und der erste Nukleinsäure-färbende Fluoreszenzfarbstoff in einem ersten Reagens enthalten sind und das erste Reagens ein Reagens zur Bestimmung einer Blutzellgruppe aus Lymphoblast und kernhaltigen roten Blutzellen ist;
ein Codesegment zum Zählen der Anzahl von Blutzellen (NL), die in der diskriminierten Zellgruppe enthalten sind;
ein Codesegment zum Diskriminieren kernhaltiger roter Blutzellen von anderen Blutzellgruppen, basierend auf zweiten Messdaten, wobei die zweiten Messdaten umgewandelt sind aus zweiten Ausgabe-Signalen von zweitem fluoreszenten Licht und zweitem vorwärts-gestreuten Licht, erhalten durch optische Messung an einer zweiten Assay-Probe, wobei die zweite Assay-Probe durch Mischen einer von der Blutprobe abgezweigten zweiten Blutprobe mit einem zweiten hämolytischen Mittel, das unterschiedlich von dem ersten hämolytischen Mittel ist, und einem zweiten Nukleinsäure-färbenden Fluoreszenzfarbstoff, wobei das zweite hämolytische Mittel ein hämolytisches Mittel zur Bestimmung von kernhaltigen roten Blutzellen ist, vorbereitet wird;
ein Codesegment zum Zählen der Anzahl kernhaltiger roter Blutzellen (NN), die in der diskriminierten Zellgruppe enthalten sind; und
ein Codesegment zum Vergleichen der Anzahl der Blutzellen (NL) und der Anzahl der kernhaltigen roten Blutzellen (NN).

## Revendications

1. Analyseur de sang (1), comprenant :
une section de fourniture d'échantillon de sang (43a) pour fournir un premier échantillon de sang et un second échantillon de sang séparé d'un échantillon de sang :
une section de préparation d'échantillon (22) pour préparer un premier échantillon de dosage en mélangeant le premier échantillon de sang, un premier agent hémolytique et un premier colorant fluorescent de coloration d'acide nucléique, et un second échantillon de dosage en mélangeant le second échantillon de sang, un second agent hémolytique différent du premier agent hémolytique et un second colorant fluorescent de coloration d'acide nucléique, dans lequel le premier agent hémolytique et le premier colorant fluorescent de coloration d'acide nucléique sont contenus dans un premier réactif, ledit premier réactif est un réactif pour détecter un groupe de cellules sanguines composé de lymphoblastes et de globules rouges nucléés, et le second agent hémolytique est un agent hémolytique pour la mesure de globules rouges nucléés ;
une cuve à circulation (231) pour assurer l'écoulement du premier échantillon de dosage et du second échantillon de dosage ;
une source de lumière (237) pour irradier un écoulement du premier échantillon de dosage ou du second échantillon de dosage dans la cuve à circulation (231) ;
une section de détection optique (243, 246, 248) pour recevoir une première fluorescence et une première lumière diffusée choisie parmi la lumière diffusée vers l'avant et la lumière diffusée vers le côté émise par le premier échantillon de dosage irradié et pour recevoir une seconde fluorescence et une seconde lumière diffusée vers l'avant provenant du second échantillon de dosage irradié ; et
un contrôleur (5) configuré pour effectuer les opérations de traitement de données comprenant :
la discrimination d'un groupe de cellules composé de lymphoblastes et de globules rouges nucléés par rapport aux autres cellules sanguines, contenues dans le premier échantillon de dosage, sur la base de la première fluorescence et de la première lumière diffusée,
le comptage des cellules discriminées contenues dans le groupe de cellules,
la discrimination des globules rouges nucléés par rapport aux autres cellules sanguines, contenues dans le second échantillon de dosage, sur la base de la seconde fluorescence et de la seconde lumière diffusée vers l'avant,
le comptage des globules rouges nucléés discriminés,
l'obtention d' informations sur la présence des lymphoblastes dans l'échantillon de sang, sur la base du nombre de cellules dans le groupe de cellules et du nombre de globules rouges nucléés, et
la délivrance des informations sur la présence des lymphoblastes dans l'échantillon de sang.

2. Analyseur de sang (1) selon la revendication 1, dans lequel :
le contrôleur (5) effectue en outre une opération de comparaison du nombre de cellules contenues dans le groupe de cellules et du nombre de globules rouges nucléés, et
l'opération d'obtention est effectuée en obtenant les informations sur la présence de lymphoblastes sur la base des résultats comparatifs.

3. Analyseur de sang (1) selon la revendication 1, dans lequel :
le contrôleur (5) effectue en outre une opération de comparaison d'une valeur donnée avec une valeur de différence entre le nombre de cellules contenues dans le groupe de cellules et le nombre de globules rouges nucléés, et
l'opération d'obtention est effectuée en obtenant les informations sur la présence de lymphoblastes sur la base des résultats comparatifs.

4. Analyseur de sang (1) selon l'une quelconque des revendications 1 à 3, dans lequel les informations sur la présence de lymphoblastes contiennent le nombre de lymphoblastes.

5. Procédé pour déterminer l'existence et la non-existence de lymphoblastes dans un échantillon de sang, le procédé comprenant :
la fourniture d'un premier échantillon de sang et d'un second échantillon de sang séparé de l'échantillon de sang ;
la préparation d'un premier échantillon de dosage en mélangeant le premier échantillon de sang avec un premier agent hémolytique et un premier colorant fluorescent de coloration d'acide nucléique, dans lequel le premier agent hémolytique et le premier colorant fluorescent de coloration d'acide nucléique sont contenus dans un premier réactif, et le premier réactif est un réactif destiné à détecter un groupe de cellules sanguines composé de lymphoblastes et de globules rouges nucléés ;
la préparation d'un second échantillon de dosage en mélangeant le second échantillon de sang avec un second agent hémolytique différent du premier agent hémolytique et un second colorant fluorescent de coloration d'acide nucléique, dans lequel le second agent hémolytique est un agent hémolytique pour la mesure de globules rouges nucléés ;
la réalisation d'une mesure optique du premier échantillon de dosage pour obtenir des premiers signaux de sortie d'une première lumière fluorescente et d'une première lumière diffusée choisie parmi la lumière diffusée vers l'avant et la lumière diffusée vers le côté ;
la réalisation d'une mesure optique du second échantillon de dosage pour obtenir des seconds signaux de sortie d'une seconde lumière fluorescente et d'une seconde lumière diffusée vers l'avant ;
la conversion des premiers signaux de sortie et des seconds signaux de sortie en premières données de mesure et secondes données de mesure, respectivement ;
la discrimination d'un groupe de cellules composé de lymphoblastes et de globules rouges nucléés par rapport à d'autres groupes de cellules de sang sur la base des premières données de mesure ;
le comptage du nombre de cellules sanguines (NL) contenues dans ledit groupe de cellules discriminé ;
la discrimination de cellules sanguines nucléées par rapport aux autres groupes de cellules sanguines sur la base des secondes donnéees de mesure ;
le comptage du nombre de globules rouges nucléés (NN) contenus dans ledit groupe de cellules discriminé ; et
la détermination du fait qu'une différence entre le nombre des cellules sanguines (NL) et le nombre de globules rouges nucléés (NN) est égale ou supérieure à une valeur de base.

6. Programme informatique (54a) incorporé à un support lisible par ordinatur (54) pour déterminer l'existence et la non-existence de lymphoblastes dans un échantillon de sang, comprenant :
un segment de code pour discriminer un groupe de cellules composé de lymphoblastes et de globules rouges nucléés par rapport aux autres groupes de cellules sanguines sur la base de premières données de mesure,
dans lequel lesdites premières données de mesure sont converties à partir de premiers signaux de sortie d'une permière lumière fluorescente et d'une première lumière diffusée choisie parmi la lumière diffusée vers l'avant et la lumière diffusée vers le côté obtenus par mesure optique d'un premier échantillon de dosage, le premier échantillon de dosage étant préparé en mélangeant un premier échantillon de sang séparé de l'échantillon de sang avec un premier agent hémolytique et un premier colorant fluorescent de coloration d'acide nucléique,
dans lequel le premier agent hémolytique et le premier colorant fluorescent de coloration d'acide nucléique sont contenus dans un premier réactif, et le premier réactif est un réactif pour détecter un groupe de cellules sanguines composé de lymphoblastes et de globules rouges nucléés ;
un segment de code pour compter le nombre de cellules sanguines (NL) contenues dans ledit groupe de cellules discriminé ;
un segment de code pour discriminer les globules rouges nucléés des autres groupes de cellules sanguines sur la base de secondes données de mesure, dans lequel lesdites secondes données de mesure sont converties à partir de seconds signaux de sortie d'une seconde lumière fluorescente et d'une seconde lumière diffusée vers l'avant obtenus par mesure optique d'un second échantillon de dosage, le second échantillon de dosage étant préparé en mélangeant un second échantillon de sang séparé de l'échantillon de sang avec un second agent hémolytique différent du premier agent hémolytique et un second colorant fluorescent de coloration d'acide nucléique, dans lequel le second agent hémolytique est un agent hémolytique pour la mesure de globules rouges nucléés ;
un segment de code pour compter le nombre de globules rouges nucléés (NN) contenus dans ledit groupe de cellules discriminé ; et
un segment de code pour comparer le nombre des cellules sanguines (NL) et le nombre des globules rouges nucléés (NN).
